# EUROPEAN PATENT SPECIFICATION

(11) **EP 3 735 591 B1**
(45) Date of publication and mention of the grant of the patent: **15.06.2022**
(21) Application number: 19700082.1
(22) Date of filing: 03.01.2019
(51) Int. Cl.: G01N 33/82

(54) **METHOD OF MEASURING THE ENDOCYTIC VITAMIN D STATUS**
VERFAHREN ZUR MESSUNG DES ENDOZYTISCHEN VITAMIN-D-STATUS
PROCÉDÉ DE MESURE DU STATUT DE LA VITAMINE D ENDOCYTIQUE

(30) Priority: 03.01.2018 DE 102018100096
(43) Date of publication of application: 11.11.2020
(73) Proprietor: Immundiagnostik AG, 64625 Bensheim (DE)
(72) Inventor: ARMBRUSTER, Franz Paul, 64270 Bobenheim-Roxheim (DE); SCHAEFER, Liliana, 60590 Frankfurt/Main (DE)
(74) Representative: Benedum, Ulrich Max
(86) International application number: PCT/EP2019/050121
(87) International publication number: WO 2019/134948

(56) References cited:
- WO-A1-2008/092917
- HYUN-JEONG KIM ET AL: "Clinical Utility of Measurement of Vitamin D-Binding Protein and Calculation of Bioavailable Vitamin D in Assessment of Vitamin D Status", ANNALS OF LABORATORY MEDICINE, vol. 37, no. 1, 1 January 2017 (2017-01-01), page 34, XP055574830, ISSN: 2234-3806, DOI: 10.3343/alm.2017.37.1.34
- Anders Nykjaer ET AL: "An Endocytic Pathway Essential for Renal Uptake and Activation of the Steroid 25-(OH) Vitamin D 3 all 25-(OH) vitamin D 3 molecules in the circulation are present in a complex with DBP. Only approximately", Cell, 19 February 1999 (1999-02-19), pages 507-515, XP055466866, Retrieved from the Internet: URL:http://www.cell.com/cell/pdf/S0092-867 4(00)80655-8.pdf
- SOFIE MALMSTROEM ET AL: "Current Assays to Determine Free 25-Hydroxyvitamin D in Serum", JOURNAL OF AOAC INTERNATIONAL, vol. 100, no. 5, 1 September 2017 (2017-09-01), pages 1323-1327, XP055574831, US ISSN: 1060-3271, DOI: 10.5740/jaoacint.17-0085 cited in the application

### SEQUENCE LISTING

<110> Immundiagnostik AG
<120> Method of measuring the endocytic vitamin D status
<130> IDK00126PC00
<150> DE102018100096.0
   <151> 2018-01-03
<160> 13
<170> PatentIn version 3.5
<210> 1
   <211> 15
   <212> PRT
   <213> Homo sapiens
<400> 1
   Asp Asn Gly Asn Cys Ile His Arg Ala Trp Leu Cys Asp Arg Asp
   1               5                   10                  15
<210> 2
   <211> 15
   <212> PRT
   <213> Homo Sapiens
<400> 2
   Gly Cys Thr His Glu Cys Val Gln Glu Pro Phe Gly Ala Lys Cys
   1               5                   10                  15
<210> 3
   <211> 5
   <212> PRT
   <213> Homo sapiens
<400> 3
   Cys Val Gln Glu Pro
   1               5
<210> 4
   <211> 5
   <212> PRT
   <213> Homo sapiens
<400> 4
   Ile His Arg Ala Trp
   1               5
<210> 5
   <211> 15
   <212> PRT
   <213> Homo sapiens
<400> 5
   Ser Asp Phe Asn Gly Gly Cys Thr His Glu Cys Val Gln Glu Pro
   1               5                   10                  15
<210> 6
   <211> 15
   <212> PRT
   <213> Homo sapiens
<400> 6
   Cys Tyr Asn Met Arg Gly Ser Phe Arg Cys Ser Cys Asp Thr Gly
   1               5                   10                  15
<210> 7
   <211> 15
   <212> PRT
   <213> Homo sapiens
<400> 7
   Phe Ser Phe Pro Cys Lys Asn Gly Arg Cys Val Pro Asn Tyr Tyr
   1               5                   10                  15
<210> 8
   <211> 391
   <212> PRT
   <213> Homo Sapiens
<400> 8
   Met Asp Arg Gly Pro Ala Ala Val Ala Cys Thr Leu Leu Leu Ala Leu
   1               5                   10                  15
   Val Ala Cys Leu Ala Pro Ala Ser Gly Gln Glu Cys Asp Ser Ala His
               20                  25                  30
   Phe Arg Cys Gly Ser Gly His Cys Ile Pro Ala Asp Trp Arg Cys Asp
           35                  40                  45
   Gly Thr Lys Asp Cys Ser Asp Asp Ala Asp Glu Ile Gly Cys Ala Trp
       50                  55                  60
   Thr Cys Gln Gln Gly Tyr Phe Lys Cys Gln Ser Glu Gly Gln Cys Ile
   65                  70                  75                  80
   Pro Asn Ser Val Trp Cys Asp Gln Asp Gln Asp Cys Asp Asp Gly Ser
                   85                  90                  95
   Asp Glu Arg Gln Asp Cys Ser Gln Ser Thr Cys Ser Ser His Gln Ile
               100                 105                 110
   Thr Cys Ser Asn Gly Gln Cys Ile Pro Ser Glu Tyr Arg Cys Asp His
           115                 120                 125
   Val Arg Asp Cys Pro Asp Gly Ala Asp Glu Asn Asp Cys Gln Tyr Pro
       130                 135                 140
   Thr Cys Glu Gln Leu Thr Cys Asp Asn Gly Ala Cys Tyr Asn Thr Ser
   145                 150                 155                 160
   Gln Lys Cys Asp Trp Lys Val Asp Cys Arg Asp Ser Ser Asp Glu Ile
                   165                 170                 175
   Asn Cys Thr Glu Ile Cys Leu His Asn Glu Phe Ser Cys Gly Asn Gly
               180                 185                 190
   Glu Cys Ile Pro Arg Ala Tyr Val Cys Asp His Asp Asn Asp Cys Gln
           195                 200                 205
   Asp Gly Ser Asp Glu His Ala Cys Asn Tyr Pro Thr Cys Gly Gly Tyr
       210                 215                 220
   Gln Phe Thr Cys Pro Ser Gly Arg Cys Ile Tyr Gln Asn Trp Val Cys
   225                 230                 235                 240
   Asp Gly Glu Asp Asp Cys Lys Asp Asn Gly Asp Glu Asp Gly Cys Glu
                   245                 250                 255
   Ser Gly Pro His Asp Val His Lys Cys Ser Pro Arg Glu Trp Ser Cys
               260                 265                 270
   Pro Glu Ser Gly Arg Cys Ile Ser Ile Tyr Lys Val Cys Asp Gly Ile
           275                 280                 285
   Leu Asp Cys Pro Gly Arg Glu Asp Glu Asn Asn Thr Ser Thr Gly Lys
       290                 295                 300
   Tyr Cys Ser Met Thr Leu Cys Ser Ala Leu Asn Cys Gln Tyr Gln Cys
   305                 310                 315                 320
   His Glu Thr Pro Tyr Gly Gly Ala Cys Phe Cys Pro Pro Gly Tyr Ile
                   325                 330                 335
   Ile Asn His Asn Asp Ser Arg Thr Cys Val Glu Phe Asp Asp Cys Gln
               340                 345                 350
   Ile Trp Gly Ile Cys Asp Gln Lys Cys Glu Ser Arg Pro Gly Arg His
           355                 360                 365
   Leu Cys His Cys Glu Glu Gly Tyr Ile Leu Glu Arg Gly Gln Tyr Cys
       370                 375                 380
   Lys His His His His His His
   385                 390
<210> 9
   <211> 452
   <212> PRT
   <213> Homo Sapiens
<400> 9
   Met Asp Arg Gly Pro Ala Ala Val Ala Cys Thr Leu Leu Leu Ala Leu
   1               5                   10                  15
   Val Ala Cys Leu Ala Pro Ala Ser Gly Gln Glu Cys Asp Ser Ala His
               20                  25                  30
   Phe Arg Cys Gly Ser Gly His Cys Glu Gln Cys Gly Leu Phe Ser Phe
           35                  40                  45
   Pro Cys Lys Asn Gly Arg Cys Val Pro Asn Tyr Tyr Leu Cys Asp Gly
       50                  55                  60
   Val Asp Asp Cys His Asp Asn Ser Asp Glu Gln Leu Cys Gly Thr Leu
   65                  70                  75                  80
   Asn Asn Thr Cys Ser Ser Ser Ala Phe Thr Cys Gly His Gly Glu Cys
                   85                  90                  95
   Ile Pro Ala His Trp Arg Cys Asp Lys Arg Asn Asp Cys Val Asp Gly
               100                 105                 110
   Ser Asp Glu His Asn Cys Pro Thr His Ala Pro Ala Ser Cys Leu Asp
           115                 120                 125
   Thr Gln Tyr Thr Cys Asp Asn His Gln Cys Ile Ser Lys Asn Val Trp
       130                 135                 140
   Cys Asp Thr Asp Asn Asp Cys Gly Asp Gly Ser Asp Glu Lys Asn Cys
   145                 150                 155                 160
   Asn Ser Thr Glu Thr Cys Gln Pro Ser Gln Phe Asn Cys Pro Asn His
                   165                 170                 175
   Arg Cys Ile Asp Leu Ser Phe Val Cys Asp Gly Asp Lys Asp Cys Val
               180                 185                 190
   Asp Gly Ser Asp Glu Val Gly Cys Val Leu Asn Cys Thr Ala Ser Gln
           195                 200                 205
   Phe Lys Cys Ala Ser Gly Asp Lys Cys Ile Gly Val Thr Asn Arg Cys
       210                 215                 220
   Asp Gly Val Phe Asp Cys Ser Asp Asn Ser Asp Glu Ala Gly Cys Pro
   225                 230                 235                 240
   Thr Arg Pro Pro Gly Met Cys His Ser Asp Glu Phe Gln Cys Gln Glu
                   245                 250                 255
   Asp Gly Ile Cys Ile Pro Asn Phe Trp Glu Cys Asp Gly His Pro Asp
               260                 265                 270
   Cys Leu Tyr Gly Ser Asp Glu His Asn Ala Cys Val Pro Lys Thr Cys
           275                 280                 285
   Pro Ser Ser Tyr Phe His Cys Asp Asn Gly Asn Cys Ile His Arg Ala
       290                 295                 300
   Trp Leu Cys Asp Arg Asp Asn Asp Cys Gly Asp Met Ser Asp Glu Lys
   305                 310                 315                 320
   Asp Cys Pro Thr Gln Pro Phe Arg Cys Pro Ser Trp Gln Trp Gln Cys
                   325                 330                 335
   Leu Gly His Asn Ile Cys Val Asn Leu Ser Val Val Cys Asp Gly Ile
               340                 345                 350
   Phe Asp Cys Pro Asn Gly Thr Asp Glu Ser Pro Leu Cys Asn Gly Asn
           355                 360                 365
   Ser Cys Ser Asp Phe Asn Gly Gly Cys Thr His Glu Cys Val Gln Glu
       370                 375                 380
   Pro Phe Gly Ala Lys Cys Leu Cys Pro Leu Gly Phe Leu Leu Ala Asn
   385                 390                 395                 400
   Asp Ser Lys Thr Cys Glu Asp Ile Asp Glu Cys Asp Ile Leu Gly Ser
                   405                 410                 415
   Cys Ser Gln His Cys Tyr Asn Met Arg Gly Ser Phe Arg Cys Ser Cys
               420                 425                 430
   Asp Thr Gly Tyr Met Leu Glu Ser Asp Gly Arg Thr Cys Lys His His
           435                 440                 445
   His His His His
       450
<210> 10
   <211> 541
   <212> PRT
   <213> Homo Sapiens
<400> 10
   Met Asp Arg Gly Pro Ala Ala Val Ala Cys Thr Leu Leu Leu Ala Leu
   1               5                   10                  15
   Val Ala Cys Leu Ala Pro Ala Ser Gly Gln Glu Cys Asp Ser Ala His
               20                  25                  30
   Phe Arg Cys Gly Ser Gly His Cys Glu Arg Cys Gly Ala Ser Ser Phe
           35                  40                  45
   Thr Cys Ser Asn Gly Arg Cys Ile Ser Glu Glu Trp Lys Cys Asp Asn
       50                  55                  60
   Asp Asn Asp Cys Gly Asp Gly Ser Asp Glu Met Glu Ser Val Cys Ala
   65                  70                  75                  80
   Leu His Thr Cys Ser Pro Thr Ala Phe Thr Cys Ala Asn Gly Arg Cys
                   85                  90                  95
   Val Gln Tyr Ser Tyr Arg Cys Asp Tyr Tyr Asn Asp Cys Gly Asp Gly
               100                 105                 110
   Ser Asp Glu Ala Gly Cys Leu Phe Arg Asp Cys Asn Ala Thr Thr Glu
           115                 120                 125
   Phe Met Cys Asn Asn Arg Arg Cys Ile Pro Arg Glu Phe Ile Cys Asn
       130                 135                 140
   Gly Val Asp Asn Cys His Asp Asn Asn Thr Ser Asp Glu Lys Asn Cys
   145                 150                 155                 160
   Pro Asp Arg Thr Cys Gln Ser Gly Tyr Thr Lys Cys His Asn Ser Asn
                   165                 170                 175
   Ile Cys Ile Pro Arg Val Tyr Leu Cys Asp Gly Asp Asn Asp Cys Gly
               180                 185                 190
   Asp Asn Ser Asp Glu Asn Pro Thr Tyr Cys Thr Thr His Thr Cys Ser
           195                 200                 205
   Ser Ser Glu Phe Gln Cys Ala Ser Gly Arg Cys Ile Pro Gln His Trp
       210                 215                 220
   Tyr Cys Asp Gln Glu Thr Asp Cys Phe Asp Ala Ser Asp Glu Pro Ala
   225                 230                 235                 240
   Ser Cys Gly His Ser Glu Arg Thr Cys Leu Ala Asp Glu Phe Lys Cys
                   245                 250                 255
   Asp Gly Gly Arg Cys Ile Pro Ser Glu Trp Ile Cys Asp Gly Asp Asn
               260                 265                 270
   Asp Cys Gly Asp Met Ser Asp Glu Asp Lys Arg His Gln Cys Gln Asn
           275                 280                 285
   Gln Asn Cys Ser Asp Ser Glu Phe Leu Cys Val Asn Asp Arg Pro Pro
       290                 295                 300
   Asp Arg Arg Cys Ile Pro Gln Ser Trp Val Cys Asp Gly Asp Val Asp
   305                 310                 315                 320
   Cys Thr Asp Gly Tyr Asp Glu Asn Gln Asn Cys Thr Arg Arg Thr Cys
                   325                 330                 335
   Ser Glu Asn Glu Phe Thr Cys Gly Tyr Gly Leu Cys Ile Pro Lys Ile
               340                 345                 350
   Phe Arg Cys Asp Arg His Asn Asp Cys Gly Asp Tyr Ser Asp Glu Arg
           355                 360                 365
   Gly Cys Leu Tyr Gln Thr Cys Gln Gln Asn Gln Phe Thr Cys Gln Asn
       370                 375                 380
   Gly Arg Cys Ile Ser Lys Thr Phe Val Cys Asp Glu Asp Asn Asp Cys
   385                 390                 395                 400
   Gly Asp Gly Ser Asp Glu Leu Met His Leu Cys His Thr Pro Glu Pro
                   405                 410                 415
   Thr Cys Pro Pro His Glu Phe Lys Cys Asp Asn Gly Arg Cys Ile Glu
               420                 425                 430
   Met Met Lys Leu Cys Asn His Leu Asp Asp Cys Leu Asp Asn Ser Asp
           435                 440                 445
   Glu Lys Gly Cys Gly Ile Asn Glu Cys His Asp Pro Ser Ile Ser Gly
       450                 455                 460
   Cys Asp His Asn Cys Thr Asp Thr Leu Thr Ser Phe Tyr Cys Ser Cys
   465                 470                 475                 480
   Arg Pro Gly Tyr Lys Leu Met Ser Asp Lys Arg Thr Cys Val Asp Ile
                   485                 490                 495
   Asp Glu Cys Thr Glu Met Pro Phe Val Cys Ser Gln Lys Cys Glu Asn
               500                 505                 510
   Val Ile Gly Ser Tyr Ile Cys Lys Cys Ala Pro Gly Tyr Leu Arg Glu
           515                 520                 525
   Pro Asp Gly Lys Thr Cys Arg His His His His His His
       530                 535                 540
<210> 11
   <211> 346
   <212> PRT
   <213> Homo sapiens
<400> 11
   Ile Pro Ala Asp Trp Arg Cys Asp Gly Thr Lys Asp Cys Ser Asp Asp
   1               5                   10                  15
   Ala Asp Glu Ile Gly Cys Ala Val Val Thr Cys Gln Gln Gly Tyr Phe
               20                  25                  30
   Lys Cys Gln Ser Glu Gly Gln Cys Ile Pro Asn Ser Trp Val Cys Asp
           35                  40                  45
   Gln Asp Gln Asp Cys Asp Asp Gly Ser Asp Glu Arg Gln Asp Cys Ser
       50                  55                  60
   Gln Ser Thr Cys Ser Ser His Gln Ile Thr Cys Ser Asn Gly Gln Cys
   65                  70                  75                  80
   Ile Pro Ser Glu Tyr Arg Cys Asp His Val Arg Asp Cys Pro Asp Gly
                   85                  90                  95
   Ala Asp Glu Asn Asp Cys Gln Tyr Pro Thr Cys Glu Gln Leu Thr Cys
               100                 105                 110
   Asp Asn Gly Ala Cys Tyr Asn Thr Ser Gln Lys Cys Asp Trp Lys Val
           115                 120                 125
   Asp Cys Arg Asp Ser Ser Asp Glu Ile Asn Cys Thr Glu Ile Cys Leu
       130                 135                 140
   His Asn Glu Phe Ser Cys Gly Asn Gly Glu Cys Ile Pro Arg Ala Tyr
   145                 150                 155                 160
   Val Cys Asp His Asp Asn Asp Cys Gln Asp Gly Ser Asp Glu His Ala
                   165                 170                 175
   Cys Asn Tyr Pro Thr Cys Gly Gly Tyr Gln Phe Thr Cys Pro Ser Gly
               180                 185                 190
   Arg Cys Ile Tyr Gln Asn Trp Val Cys Asp Gly Glu Asp Asp Cys Lys
           195                 200                 205
   Asp Asn Gly Asp Glu Asp Gly Cys Glu Ser Gly Pro His Asp Val His
       210                 215                 220
   Lys Cys Ser Pro Arg Glu Trp Ser Cys Pro Glu Ser Gly Arg Cys Ile
   225                 230                 235                 240
   Ser Ile Tyr Lys Val Cys Asp Gly Ile Leu Asp Cys Pro Gly Arg Glu
                   245                 250                 255
   Asp Glu Asn Asn Thr Ser Thr Gly Lys Tyr Cys Ser Met Thr Leu Cys
               260                 265                 270
   Ser Ala Leu Asn Cys Gln Tyr Gln Cys His Glu Thr Pro Tyr Gly Gly
           275                 280                 285
   Ala Cys Phe Cys Pro Pro Gly Tyr Ile Ile Asn His Asn Asp Ser Arg
       290                 295                 300
   Thr Cys Val Glu Phe Asp Asp Cys Gln Ile Trp Gly Ile Cys Asp Gln
   305                 310                 315                 320
   Lys Cys Glu Ser Arg Pro Gly Arg His Leu Cys His Cys Glu Glu Gly
                   325                 330                 335
   Tyr Ile Leu Glu Arg Gly Gln Tyr Cys Lys
               340                 345
<210> 12
   <211> 406
   <212> PRT
   <213> Homo sapiens
<400> 12
   Glu Gln Cys Gly Leu Phe Ser Phe Pro Cys Lys Asn Gly Arg Cys Val
   1               5                   10                  15
   Pro Asn Tyr Tyr Leu Cys Asp Gly Val Asp Asp Cys His Asp Asn Ser
               20                  25                  30
   Asp Glu Gln Leu Cys Gly Thr Leu Asn Asn Thr Cys Ser Ser Ser Ala
           35                  40                  45
   Phe Thr Cys Gly His Gly Glu Cys Ile Pro Ala His Trp Arg Cys Asp
       50                  55                  60
   Lys Arg Asn Asp Cys Val Asp Gly Ser Asp Glu His Asn Cys Pro Thr
   65                  70                  75                  80
   His Ala Pro Ala Ser Cys Leu Asp Thr Gln Tyr Thr Cys Asp Asn His
                   85                  90                  95
   Gln Cys Ile Ser Lys Asn Trp Val Cys Asp Thr Asp Asn Asp Cys Gly
               100                 105                 110
   Asp Gly Ser Asp Glu Lys Asn Cys Asn Ser Thr Glu Thr Cys Gln Pro
           115                 120                 125
   Ser Gln Phe Asn Cys Pro Asn His Arg Cys Ile Asp Leu Ser Phe Val
           130                 135                 140
   Cys Asp Gly Asp Lys Asp Cys Val Asp Gly Ser Asp Glu Val Gly Cys
   145                 150                 155                 160
   Val Leu Asn Cys Thr Ala Ser Gln Phe Lys Cys Ala Ser Gly Asp Lys
                   165                 170                 175
   Cys Ile Gly Val Thr Asn Arg Cys Asp Gly Val Phe Asp Cys Ser Asp
               180                 185                 190
   Asn Ser Asp Glu Ala Gly Cys Pro Thr Arg Pro Pro Gly Met Cys His
           195                 200                 205
   Ser Asp Glu Phe Gln Cys Gln Glu Asp Gly Ile Cys Ile Pro Asn Phe
       210                 215                 220
   Trp Glu Cys Asp Gly His Pro Asp Cys Leu Tyr Gly Ser Asp Glu His
   225                 230                 235                 240
   Asn Ala Cys Val Pro Lys Thr Cys Pro Ser Ser Tyr Phe His Cys Asp
                   245                 250                 255
   Asn Gly Asn Cys Ile His Arg Ala Trp Leu Cys Asp Arg Asp Asn Asp
               260                 265                 270
   Cys Gly Asp Met Ser Asp Glu Lys Asp Cys Pro Thr Gln Pro Phe Arg
           275                 280                 285
   Cys Pro Ser Trp Gln Trp Gln Cys Leu Gly His Asn Ile Cys Val Asn
       290                 295                 300
   Leu Ser Val Val Cys Asp Gly Ile Phe Asp Cys Pro Asn Gly Thr Asp
   305                 310                 315                 320
   Glu Ser Pro Leu Cys Asn Gly Asn Ser Cys Ser Asp Phe Asn Gly Gly
                   325                 330                 335
   Cys Thr His Glu Cys Val Gln Glu Pro Phe Gly Ala Lys Cys Leu Cys
               340                 345                 350
   Pro Leu Gly Phe Leu Leu Ala Asn Asp Ser Lys Thr Cys Glu Asp Ile
           355                 360                 365
   Asp Glu Cys Asp Ile Leu Gly Ser Cys Ser Gln His Cys Tyr Asn Met
   370                 375                 380
   Arg Gly Ser Phe Arg Cys Ser Cys Asp Thr Gly Tyr Met Leu Glu Ser
   385                 390                 395                 400
   Asp Gly Arg Thr Cys Lys
                   405
<210> 13
   <211> 495
   <212> PRT
   <213> Homo sapiens
<400> 13
   Glu Arg Cys Gly Ala Ser Ser Phe Thr Cys Ser Asn Gly Arg Cys Ile
   1               5                   10                  15
   Ser Glu Glu Trp Lys Cys Asp Asn Asp Asn Asp Cys Gly Asp Gly Ser
               20                  25                  30
   Asp Glu Met Glu Ser Val Cys Ala Leu His Thr Cys Ser Pro Thr Ala
           35                  40                  45
   Phe Thr Cys Ala Asn Gly Arg Cys Val Gln Tyr Ser Tyr Arg Cys Asp
       50                  55                  60
   Tyr Tyr Asn Asp Cys Gly Asp Gly Ser Asp Glu Ala Gly Cys Leu Phe
   65                  70                  75                  80
   Arg Asp Cys Asn Ala Thr Thr Glu Phe Met Cys Asn Asn Arg Arg Cys
                   85                  90                  95
   Ile Pro Arg Glu Phe Ile Cys Asn Gly Val Asp Asn Cys His Asp Asn
               100                 105                 110
   Asn Thr Ser Asp Glu Lys Asn Cys Pro Asp Arg Thr Cys Gln Ser Gly
           115                 120                 125
   Tyr Thr Lys Cys His Asn Ser Asn Ile Cys Ile Pro Arg Val Tyr Leu
       130                 135                 140
   Cys Asp Gly Asp Asn Asp Cys Gly Asp Asn Ser Asp Glu Asn Pro Thr
   145                 150                 155                 160
   Tyr Cys Thr Thr His Thr Cys Ser Ser Ser Glu Phe Gln Cys Ala Ser
                   165                 170                 175
   Gly Arg Cys Ile Pro Gln His Trp Tyr Cys Asp Gln Glu Thr Asp Cys
               180                 185                 190
   Phe Asp Ala Ser Asp Glu Pro Ala Ser Cys Gly His Ser Glu Arg Thr
           195                 200                 205
   Cys Leu Ala Asp Glu Phe Lys Cys Asp Gly Gly Arg Cys Ile Pro Ser
       210                 215                 220
   Glu Trp Ile Cys Asp Gly Asp Asn Asp Cys Gly Asp Met Ser Asp Glu
   225                 230                 235                 240
   Asp Lys Arg His Gln Cys Gln Asn Gln Asn Cys Ser Asp Ser Glu Phe
                   245                 250                 255
   Leu Cys Val Asn Asp Arg Pro Pro Asp Arg Arg Cys Ile Pro Gln Ser
               260                 265                 270
   Trp Val Cys Asp Gly Asp Val Asp Cys Thr Asp Gly Tyr Asp Glu Asn
           275                 280                 285
   Gln Asn Cys Thr Arg Arg Thr Cys Ser Glu Asn Glu Phe Thr Cys Gly
       290                 295                 300
   Tyr Gly Leu Cys Ile Pro Lys Ile Phe Arg Cys Asp Arg His Asn Asp
   305                 310                 315                 320
   Cys Gly Asp Tyr Ser Asp Glu Arg Gly Cys Leu Tyr Gln Thr Cys Gln
                   325                 330                 335
   Gln Asn Gln Phe Thr Cys Gln Asn Gly Arg Cys Ile Ser Lys Thr Phe
               340                 345                 350
   Val Cys Asp Glu Asp Asn Asp Cys Gly Asp Gly Ser Asp Glu Leu Met
           355                 360                 365
   His Leu Cys His Thr Pro Glu Pro Thr Cys Pro Pro His Glu Phe Lys
       370                 375                 380
   Cys Asp Asn Gly Arg Cys Ile Glu Met Met Lys Leu Cys Asn His Leu
   385                 390                 395                 400
   Asp Asp Cys Leu Asp Asn Ser Asp Glu Lys Gly Cys Gly Ile Asn Glu
                   405                 410                 415
   Cys His Asp Pro Ser Ile Ser Gly Cys Asp His Asn Cys Thr Asp Thr
               420                 425                 430
   Leu Thr Ser Phe Tyr Cys Ser Cys Arg Pro Gly Tyr Lys Leu Met Ser
           435                 440                 445
   Asp Lys Arg Thr Cys Val Asp Ile Asp Glu Cys Thr Glu Met Pro Phe
       450                 455                 460
   Val Cys Ser Gln Lys Cys Glu Asn Val Ile Gly Ser Tyr Ile Cys Lys
   465                 470                 475                 480
   Cys Ala Pro Gly Tyr Leu Arg Glu Pro Asp Gly Lys Thr Cys Arg
                   485                 490                 495

## Description

### FIELD OF THE INVENTION

The present application relates to assays involving biological materials of a specific nature and in particular to an assay for measuring the endocytable vitamin D concentration in a biological sample of a subject, notably blood or serum, and for detection or diagnosis of diseases or conditions related to vitamin D status in blood or serum (G01N 2333/00, G01N2800/00)

### BACKGROUND OF THE INVENTION

The metabolic pathway leading to the synthesis of active vitamin D involves three reactions that occur in different tissues. In humans the synthesis is initiated in the skin with a UV light-mediated cleavage to produce cholecalciferol (vitamin D₃, VD₃). The other vitamin D isomer "ergocalciferol" (vitamin D₂, VD₂) occurs in plants and is taken up with the food. Both vitamin D isomers are metabolized in the liver to 25-hydroxyvitamin D [25(OH)D] which is also the major circulatory form (prohormone). This second step is catalyzed by a cytochrome P450 enzyme, a NAPH-hemoprotein reductase, while the identity of the hepatic 25-hydroxylase still require more elucidation. The 25(OH)D then becomes 1a-hydroxylated in the kidney to 1α,25-dihydroxyvitamin D or calcitriol which is the physiologically active form (D-hormone). Calcitriol regulates the absorption of calcium in the intestines, the mineralization of the bones, the differentiation of osteoblasts, the synthesis of bone matrix and neuromuscular functions. It is common medical knowledge that 25(OH)D levels in serum lower than 15 ng per mL serum (37.5 nmol/L) cause a rise of the parathyroid hormone level and leads to an increase in bone resorption (Chapuy MC et al. in J Clin Endocrinol Metab 1996; 81:1129-33). A vitamin D deficiency may be caused by gastro-intestinal diseases, liver dysfunction, malabsorption drug-induced heightened metabolism, genetic defects or insufficient exposure to sunlight. Vitamin D deficiency is a known risk factor for senile osteoporosis. Generally, a deficiency of less than 5 ng 25(OH)D per mL serum (12.5 nmol/L) is regarded severe, causing rickets in children and osteomalacia in adults (Scharla et al. Exp Clin Endocrinol. Diabetes, 1996, 104:289-292). Excess of vitamin D due to overdosing causes hypercalcemia. It is not clear whether there is a definitive difference between the effects of VD₂ and VD₃ or whether they are equally effective in the raising of serum 25(OH)D, particularly at lower doses of vitamin D (cf: Tripkovic L et al, Am J Clin Nutr. 2012 Jun; 95(6): 1357-1364; Am J Clin Nutr. 2017 Aug;106(2):481-490).

Vitamin D binding protein (DBP) is a member of the albuminoid superfamily. The 56-58 kDa glucoprotein can bind vitamin D metabolites as well as fatty acids and other endotoxins. It is thought that DBP acts as a reservoir in situations of deficiency, increasing the half-life of vitamin D, but also protects against vitamin D intoxication. The concentration of DBP in blood is stably maintained within a relatively narrow range (323-460 mg/L (5.52-7.93 mol/L)) in normal subjects, even in pathological conditions and disorders of the calcium metabolism. Except in pregnancy, no high DBP concentrations have been observed. DBP in serum can be measured by numerous methods, including immunoturbidimetry (Hamashima et al, Clinica Chimica Acta 321 (2002) 23-28). DBP is known to produce chemokinetic effects on neutrophil granulocytes, activate macrophages and sequester actin upon tissue damage.

The DBP level in serum is about 20-fold higher than of vitamin D and therefore 2 to 5% only will be occupied by vitamin D metabolites. For measurement, the vitamin D metabolites are released from DBP by enzymatic digestion, denaturing and/or ligand displacement (EP 2 126 586 B1, WO 99/67211; EP 0 753 743, WO 2004/063704). The releasing agents include all types of detergents and surfactants (EP 2 955 516 B1) as well as structural analogs of vitamin D such as warfarin, salicylic compounds, certain sulfonic acids, toluene sulfonic acids, naphthalene sulfonic acid, anilinonaphthalene sulfonic acids, etc (WO 03/023391). The release of vitamin D from DBP is a decisive step in most laboratory procedures, in particular as the vitamin D metabolites are hydrophobic and cholesterol-like, bound by numerous serum proteins, and so their quantitative determination is technically difficult and results open to interpretation. The confusion as to the vitamin D status gave rise to collaborative initiative led by the Office of Dietary Supplements of the U.S. National institutes of Health and there have recently been proposals for assessing the vitamin D status via a determination of the "free 25(OH)D" in serum, say the 25(OH)D not bound by DBP or albumin (for review: The Importance of 25-Hydroxyvitamin D Assay Standardization and the Vitamin D Standardization Program. J AOAC Int. 2017, 100(5):1223-1224; and Malmstroem S et al, J AOAC Int. 2017, 100(5):1323-7). Further background art is contained in Hyun-Jeong Kim et al, Clinical Utility of Measurement of Vitamin D-Binding Protein and Calculation of Bioavailable Vitamin D in Assessment of vitamin D Status, Ann Lab Med 2017, 37; 34-38 and in the article of Nykjaer A et al in An endocytic pathway essential for the renal uptake and activation of the steroid 25-(OH)-vitamin D3, Cell 1999, 96:507-515.

Calcium and phosphorus are essential minerals required for many critical biologic functions including cell signaling, energy metabolism, skeletal growth and integrity. Calcium and phosphate homeostasis are maintained primarily by regulation of epithelial calcium and phosphate cotransport in the kidney and intestine, processes that are tightly regulated by hormones including calcitriol, fibroblast growth factor 23 (FGF23) and parathyroid hormone (PTH). In patients with chronic kidney disease (CKD), as renal function declines, disruption of feedback loops between these hormones have adverse consequences on several organ systems, including the skeleton, heart and vascular system. Complications include vascular calcification, stroke, skeletal fracture and increased risk of death. Increased FGF23 and PTH concentrations, and vitamin D deficiency contributes to the pathogenesis. Therefore, treatment of patients is focused on restoring the feedback loops to maintain normal calcium and phosphate balance to prevent skeletal and cardiovascular complications. Recent evidence is further linking the vitamin D status to disorders such as cancer, diabetes, depression, etc. leading to a higher demand of routine testing for vitamin D levels even in healthy patients. The importance of the vitamin D status in research and health cannot therefore be overstated and clinical laboratories are confronted with the challenge of increasing test numbers and the need to identify persons truly suffering from a low vitamin D status. The state of the art represents a problem.

### SUMMARY OF THE INVENTION

The application provides an assay and a method of measuring the effective vitamin D level (including the vitamin D metabolites of the storage form) in a sample of bodily fluid in the presence of vitamin D binding protein (DBP), comprising the steps of a) contacting said sample with megalin/LRP2 (*low-density lipoprotein related protein* 2) and/or a soluble fragment thereof under binding conditions to form a complex containing DBP, vitamin D or a metabolite thereof and megalin/LRP2 or a fragment thereof; b) determining the amount of DBP:vitamin D and/or any one of its components; and c) relating the amount of megalin-bound complex of DBP:vitamin D to the effective vitamin D status in said subject.

In a preferred embodiment, the assay and method comprise the use of a fragment of megalin which binds none of the other ligands of megalin/LRP2, and/or a fusion protein with said fragment of megalin which can bind the DBP:VD complex. A preferred embodiment of said method comprises a differential measurement of hydroxylated cholecalciferol (25-hydroxyvitamin D₃) in the presence of hydroxylated ergocalciferol (25-hydroxyvitamin D₂), 24,25-dihydroxyvitamin D₂, and/or 24,25-dihydroxyvitamin D₃.

The disclosure encompasses the use of the endogenous DBP present in serum but may include the addition of DBP to obtain a standard concentration of DBP in the test samples. The disclosure may further encompass contacting additionally said sample with cubilin and/or a soluble fragment thereof as cubilin is known to facilitate the endocytic process (Nykjaer A, et al Cubilin dysfunction causes abnormal metabolism of the steroid hormone 25(OH) vitamin D3. PNAS U.S.A. (2001) 98(24):13895-900 [PUBMED:11717447].

In one embodiment, the amount of DBP:VD bound by megalin is determined by turbidimetry or nephelometry or, notably, by an immunoassay for DBP. In another embodiment, the megalin and/or said soluble fragment thereof may be bound to particles or beads having diameters ranging from 50 to 200 nm. Alternatively, the disclosure teaches for the sake of completion an immunoassay selected from the group ELISA (enzyme-linked immunosorbent assay), RIA (radioimmunoassay), FIA (fluorescence immunoassay), LIA (luminescence immunoassay), or ILMA.

In another embodiment the method may comprise the steps of (a) providing a defined amount of megalin and/or a fragment thereof coupled to a solid phase; (b) contacting the sample with a solid phase; (c) creating conditions to allow binding of megalin and/or a fragment thereof to the complex formed between DBP and vitamin D metabolite, wherein DBP alone is not bound by megalin and/or a fragment thereof; and washing the solid phase; (e) providing an antibody recognizing the complex of DBP:VD or, facultatively, megalin or a soluble fragment thereof; (f) contacting said megalin and/or fragment thereof bound to DBP:VD with an anti-DBP-antibody, and optionally, immobilizing the complex on the solid phase; and (g) determining the amount of antibody bound to the solid phase, and quantitating the vitamin D status in plasma or serum by correlation with a reference.

The disclosure also provides a test kit which comprises an antibody specific for DBP and a binding partner comprising megalin or a fragment thereof. The antibody may be one binding to DBP or vitamin D metabolite in said complex. In another embodiment, the test kit may comprise nanoparticles having bound megalin and/or soluble fragments thereof.

The stated object is achieved because the disclosure provides a method for direct quantitative determination of the effective vitamin D status in serum or plasma. The status is based on the major circulating vitamin D metabolite which is ready for endocytosis and internalization into cells of the kidney, or into cells having an endocytic megalin transport pathway, so that the prohormone (25(OH)D) will be 1a-hydroxylated to the physiologically active D-hormone (calcitriol). As ergocalciferol and cholecalciferol are both bound by DBP but the complex of DBP:VD₃ is predominantly bound by megalin, the disclosed method provides a status of prohormone capable of becoming the active hormone. Vitamin D metabolites such as 24,25(dihydroxy)vitamin-D₃, while they can still form a complex with DBP, are either much less bound by megalin or their concentration is too low in the circulation to impact the determined effective vitamin D status.

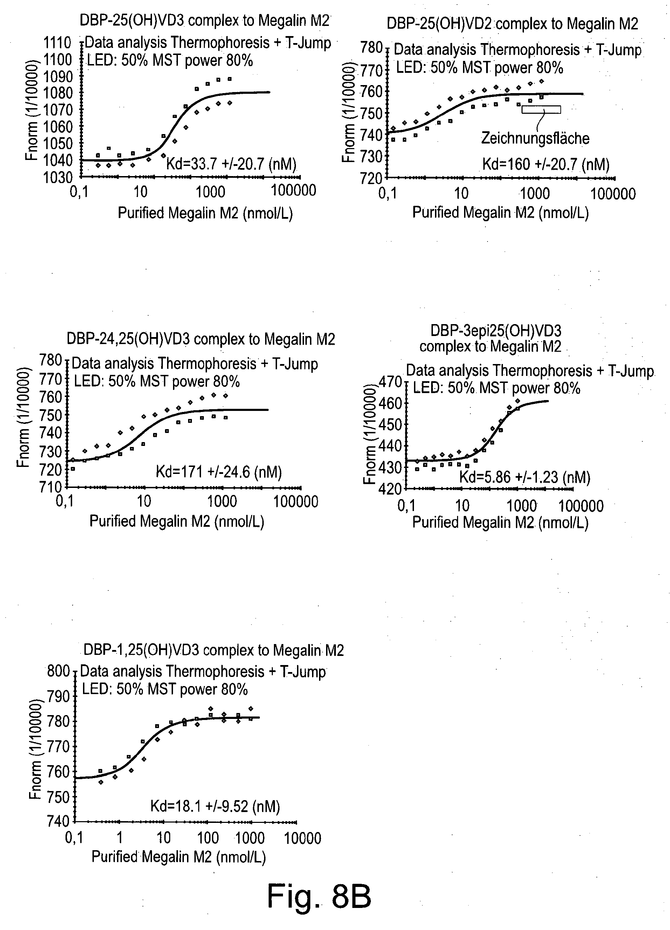

The DBP:VD₃ complex only is endocytosed by the megalin cubulin pathway into cells where the P450 1a-hydroxylase (Cyp27B1) is located on the outside of the mitochondrial membrane. Consequently, other vitamin D metabolites will either not become activated or their concentration in the circulation is too low for being relevant with respect to the measured vitamin D status. This applies in particular to 25-hydroxylated ergocalciferol [25(OH)D₂] which complex with DBP is not or much less bound by megalin (see **Fig.** 8B). The present disclosure therefore contradicts the free hormone hypothesis according to which only the non-protein-bound fraction (the free fraction) of vitamin D metabolites can enter cells and exert biologic effects.

The determination of the effective vitamin D status can be done in aqueous solution despite of the lipophilic nature of the vitamin D metabolites. There is no need for releasing the vitamin D metabolites from their binding partners (DBP, albumin, proteins of the albuminoid superfamily, etc.) as the method is based on the measurement of the complex of DBP:VD which will be bound or discriminated by megalin or a soluble fragment thereof. A reliable discrimination of vitamin binding protein having bound vitamin D is provided. Megalin or fragments thereof are used to discern the vitamin D status based on the endocytic DBP:VD complex which we submit represents the solely bioactivatable prohormone in the circulation.

Soluble megalin can be produced in mammalian cells by recombinant methods and purified by affinity chromatography. Megalin fragments soluble in aqueous solution (serum, plasma) are preferred as they allow conditions for the formation of DBP:VD complex close to physiological.

As disclosed, there is no need to release vitamin D from its binding partners as by prior art methods. Thus, there is no need for halogenated solvents, tensids and surfactants (PFOA, CTAB) which makes the method environmentally friendly. The lack of preanalytical purification and preparation steps further allows a direct determination of the effective vitamin D status immediately after sample collection. The concept of determining the formation of said megalin-bound complex can easily be adapted to platforms such as ELISA, turbidimetry and nephelometry. The proteinaceous components of said complex can be reliably quantified.

The present disclosure is further in conformity with clinical reports that 25-hydroxyvitamin D₃ is more effective than 25-hydroxyvitamin D₂. The disclosure provides an effective status on basis of the endocytable prohormone which will be available for activation. While the 25-hydroxylated vitamin D₂ and D₃ isomers are equally bound by the DBP the provided method makes use of the different binding affinity of megalin to DBP when in interaction with 25-hydroxyvitamin D₃. The different binding affinity is striking. It is submitted that a fundamental biological mechanism has been discovered which physiological relevance cannot be overlooked. The present method allows a discrimination of the activatable circulatory vitamin D metabolites and therefore provides a status of the readily activatable vitamin D metabolite (prohormone). With the information provided, vitamin D supplementation therapies can be adapted to the true physiological status of the subject, avoiding toxicity or inefficient therapies.

The principles of invention will now be described by reference to its advantages, representative examples and drawings which shall, however, not limit the gist of the invention which can be derived from the disclosure contained in the claims.

### BRIEF DESCRIPTION OF THE DRAWINGS

In the accompanying drawings,
- **Figs. 1A-C**: are schematic representations showing **(A)** potential binding sites of human DBP (dimer) to megalin as predicted by PepSite 2 (Trabuco LG et al, PepSite: prediction of peptide-binding sites from protein surfaces in Nucleic Acids Res. 2012; 40(Web Server issue):W423-426); **(B)** the interaction between megalin/LRP2, cubulin and their known ligands on the outside of the luminal plasma membrane; and (C) the pathways from vitamin D via the prohormone (25(OH)D to the active hormone (1,25(OH)₂D - calcitriol) as well as excretion pathways;

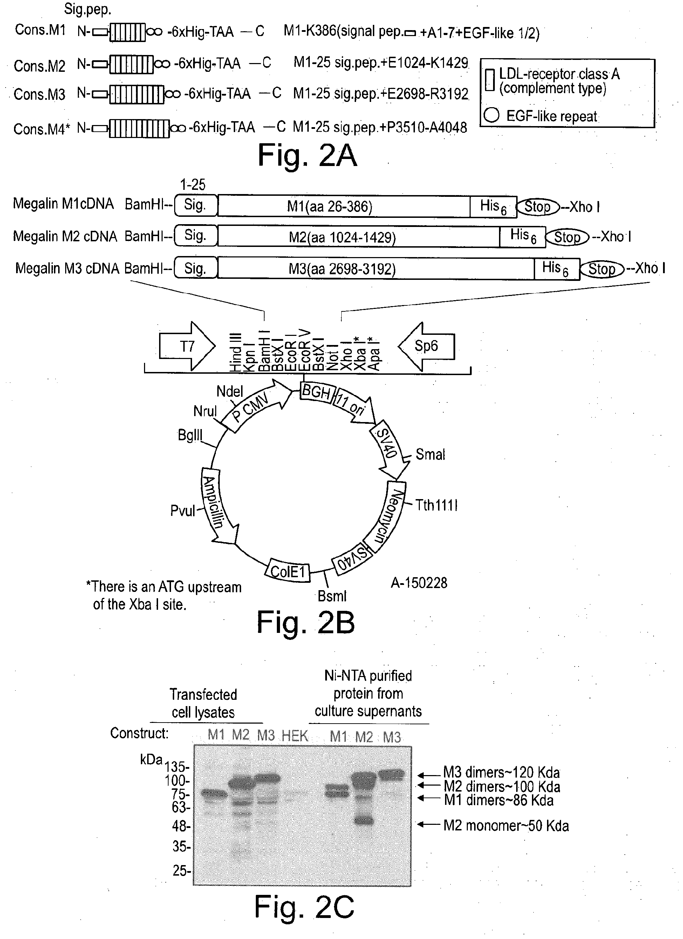

- **Figs. 2A-C**: are schematic representations of **(A)** chosen megalin fragments tested for binding of vitamin D binding protein: Cons M1: M1-K386 (signal peptide + A1-7 + EGF- like ½); M2: (M1-25 signal peptide + E1024-K1429); M3: (M1-25 signal peptide + E2698-R3192; M4: (M1-25 signal peptide + P3510-A4048 - not further examined); **(B)** the cDNA constructs of megalin fragments (M1, M2, and M3) cloned into expression vector pcDNA3 (Invitrogen, San Diego, CA); and **(C)** Western blots of secreted megalin fragments (M1-M3 dimers) and M2 monomer: HEK cell lysates and after Ni-NTA affinity purification from culture supernatants;
- **Figs. 3A,B**: show Western blots of **(A)** megalin fragments from transfected HEK cell lysates (M1, M2, M3 + control/Non; -20 µg) and **(B)** co-immunoprecipitations of the megalin-bound (M1, M2, M3) DBP (0,5 µg) in the presence or absence of VD₃ (0,2 µg) using anti-His-antibody (WB staining: anti-DBP Ab);
- **Figs. 4A,B**: show Western blots **(A)** of Ni-NTA purified megalin fragments (M1, M2 and M3 + Anti-IgG AB as control; ~3 µg) and **(B)** co-immunoprecipitations of the complex of megalin bound DBP (2 µg) in the presence or absence of VD₃ (0,5 µg) using an anti-His-antibody (WB staining: anti-DBP Ab);
- **Figs. 5A,B**: show (A) microscale thermophoresis analyses of megalin fragments M1, M2 and M3 and DBP (50 nM) in the presence or absence of VD₃ (50nM); and **(B)** the effect of the VD₃ concentration on the affinity analysis;
- **Figs. 6A,B**: show Western blots (A) of co-immunoprecipitated DBP from different human sera or plasma using purified megalin fragment M1 (3 µg); and a bar diagram (B) showing the results of an ELISA for total DBP and formed DBP/VD₃ bound by surface-coated megalin fragment (M1 or M2) ;
- **Figs. 7A-C**: are graphs (A) showing the effect of added purified DBP on the formation of M2-megalin-bound DBP:25(OH)D₃ in a sample of human serum of a subject (RMS); and (B) the correlation between 25(OH)D₃ serum levels and the binding of DBP:25(OH)D₃ to megalin M2 fragment in human serum; and (C) the correlation between 25(OH)D₃ level and ternary complex DBP/25(OH)D₃/M2 and the same for the ternary complexes with 25(OH)D₂ and 24,25(OH)VD, respectively;
- **Fig. 8A,B**: show (A) microscale thermophoresis analyses of DBP and various 25(OH)D metabolites: (25(OH)D₃, 25(OH)D₂, 24,25(OH)D; and (B) microscale thermophoresis analyses of DBP:VD-metabolite when bound by megalin M2 fragment;
- Fig. 9A,B: are graphs showing the linearity range of megalin-bound DBP:25(OH)D 3 and in the presence of varying concentrations of 24,25(OH)VD and 25(OH)VD 2 at high concentrations (up to 50 ng/mL)
- Fig. 10A,B: are graphs showing the linearity range of megalin-bound DBP:25(OH)D 3 and in the presence of varying concentrations of 24,25(OH)VD and 25(OH)VD 2 for low concentrations (up to 19 ng/mL).

### DETAILED DESCRIPTION OF THE INVENTION

The instant description provides a method of determining the amount of biologically effective vitamin D in a sample of bodily fluid from a subject. The method comprises the steps of contacting said sample with DBP and megalin or a functional fragment thereof under binding conditions to form a complex of DBP:vitamin D (or a metabolite of vitamin D) which will be specifically bound by megalin, or a functional fragment thereof, to form a ternary complex comprising DBP:vitamin D:megalin. The amount of formed ternary complex in said sample of bodily fluid can then be correlated to the biologically effective amount of vitamin D in said sample of bodily fluid.

In this context, the term "biologically effective vitamin D" or "effective vitamin D" defines and comprises all structural vitamin D molecules which form a complex with DBP that is recognized and bound by megalin or a functional fragment thereof to form a ternary complex. Those structural vitamin D molecules therefore comprise not only vitamin D (chole- and ergocalciferol) but also the 25-hydroxylated vitamin D metabolites, including the respective epimers. For many years, emphasis has been on measuring total levels of 25-hydroxyvitamin D [25(OH)D]. As the measured values were not consistent with physiologies there has recently been hypothesized that "free 25(OH)D" is potentially better a marker of the vitamin D status. The proposed assessment of "free 25(OH)D" however relies on calculations using levels of total 25(OH)D, albumin, and DBP and on the assumption of a constant affinity of DBP for the vitamin D metabolites. This hypothesis works with the assumption that the lipophilic vitamin D metabolites can passively diffuse through cell membranes and that serum DBP and albumin would decrease the readily available amount of vitamin D and the vitamin D status. The determination of the "free DBP-unbound fraction of 25(OH)D" shall give the "actual free and available vitamin D status".

The vitamin D status is not only essential for normal kidney function and bone health but can also be linked to cancer development and some autoimmune diseases. Given the impact of the vitamin D status on human health, reliable methods are required for clinical practice.

The concentration of total "25-hydroxyvitamin D" in serum is mostly used in clinical practice but the discussion on the amount or proportion of "free or available" or hidden 25-hydroxy vitamin D" cannot be ignored. However, this hypothesis does not consider that epithelial cells and cells of other tissues (renal tubules, parathyroid gland, placenta etc.) have an endocytic pathways which enable the endocytic internalization of DBP-bound vitamin D metabolites. 25(OH)D is the key form of the prohormone for uptake and innercellular conversion to calcitriol. The vitamin D metabolites are mainly transported and bound in the circulation by the vitamin D binding protein. With prior art measurement methods, the amount of vitamin D metabolites that can become activated is not known and even less known is the amount of prohormone available for endocytic internalization and 1α-hydroxylation to the active D--hormone.

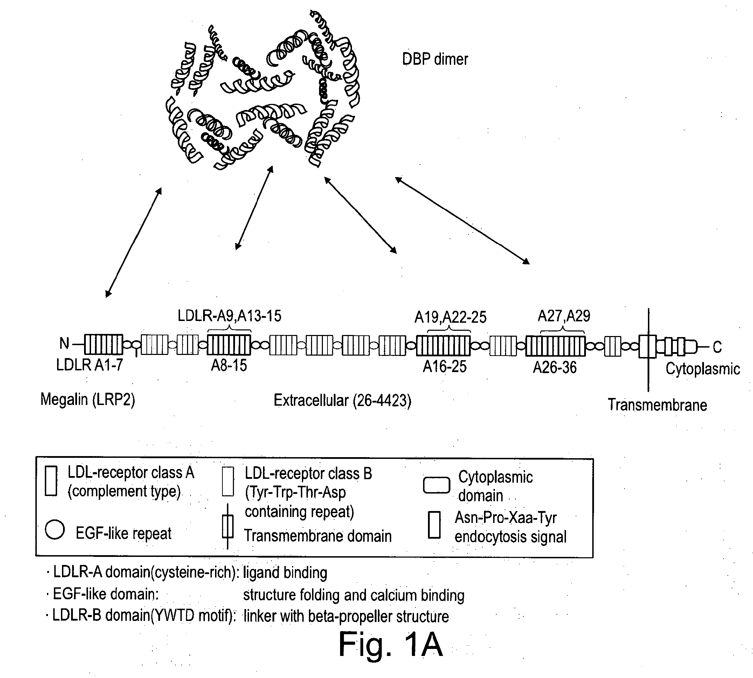

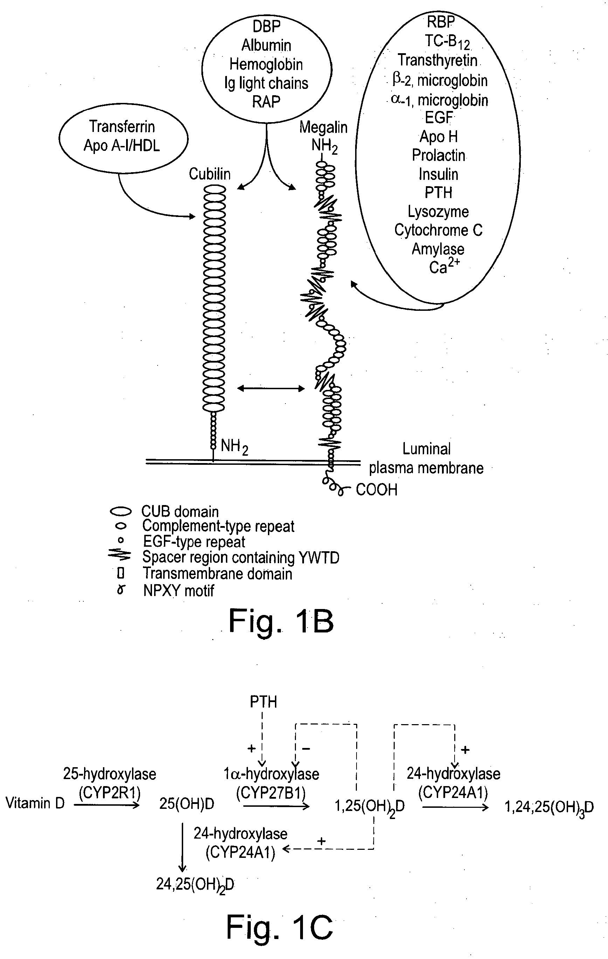

The present method makes use of that a ternary complex of vitamin D, DBP and megalin must form before the vitamin D metabolite can become internalized by endocytosis, either directly or following interaction with cubilin (cf. **Fig. 1A-C).** It is well established that vitamin D (cholecalciferol or ergocalciferol) is first hydroxylated in its 25-position to the prohormone in the liver and then present in the circulation. It also well established that the active D-hormone [1,25(OH)₂D] is synthesized by the enzyme 1α-hydroxylase (Cyp27B1 - cytochrome P450) which is located within cells on the outside of the mitochondrial membrane (cf **Fig.** 1C). The 25-hydroxylated vitamin D metabolite must be first transported from the liver to the respective cells in the kidney. The kidney is the major source of calcitriol (1,25(OH)₂D) but also extrarenal cells, including lymphocytes, macrophages, keratinocytes, and cells of the parathyroid gland and pancreas can generate calcitriol.

It is known that 25-hydroxyvitamin D₃ is 300% more effective than 25-hydroxyvitamin D₂. In line therewith, the present disclosure provides a discrimination of endocytable DBP, say DBP having bound a vitamin D₃ metabolite. Megalin or fragments thereof are used to discern the physiologically activatable vitamin D₃ metabolite. Recombinant megalin fragments can be produced in mammalian cell lines and purified by affinity chromatography. A direct and fast determination of a vitamin D status is provided and there is no need for any additional pre-treatment or sample preparation. The assay's time-scale is, thus, reduced while providing physiological accurate and valid readings. The disclosed approach can easily be adapted to different platforms such as ELISA, turbidimetry and nephelometry.

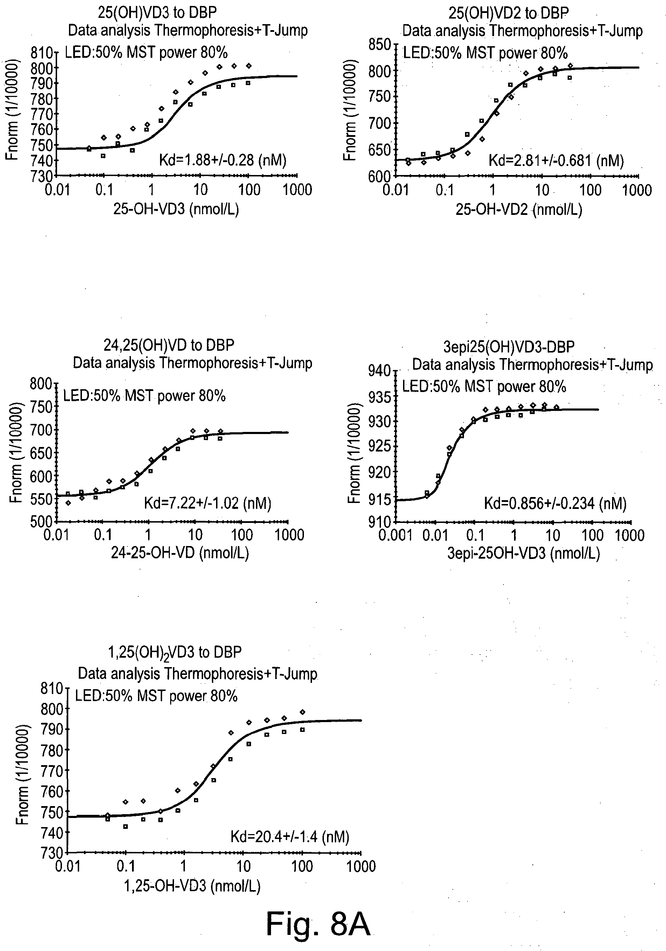

The novel vitamin D status corresponds to the physiologically activated, endocytable vitamin D, in particular, to the 25-hydroxyvitamin D₃ in serum or plasma. The concentrations of the other endocytable vitamin D metabolites are much lower. While the 25-hydroxylated vitamin D₂ and D₃ isomers are equally bound by DBP (**Fig. 8A**), the higher binding affinity of megalin to DBP:25(OH)VD₃ compared to DBP:25(OH)VD₂ and 24,25-hydroxyvitamin D is striking. A special role seems to be taken by the C3-epimer of 25-hydroxyvitamin D₃ [3-epi-25(OH)D₃], for which there is a near-total lack of data regarding its clinical significance. Although little is known regarding the *in vivo* importance of 3-epi-25(OH)D₃, clinical laboratories face the decision of whether or not to include 3-epi-25(OH)D₃ in the measurement of the vitamin D status. The data in Figs. 8A,B show that the described method can adequately detect and resolve the C-3 epimeric form of 25(OH)D₃. Therefore, the present method allows a distinction for determining the most potent vitamin D metabolites and, in turn, provides a more accurate and physiologically valid vitamin D status which does not include the less active forms. With the information provided by the present disclosure, vitamin D supplementation therapies can therefore be adapted to the physiological status of the subject, avoiding toxicity or inefficient therapies.

In a preferred embodiment, the method comprises the use of a soluble fragment of megalin and/or a fusion of said soluble megalin fragment which binds the complex of DBP:VD metabolite but none or less of the numerous other ligands of LPR2/megalin. More precisely, a soluble fragment of megalin which has no affinity for albumin or anti-DBP antibody (no Ab cross-reactivity!). Said embodiment may comprise a surface-bound DBP-binding megalin fragment or a fusion protein thereof which contains an epitope comprising :
SEQ ID NO: 01
   **-D-N-G-N-C-I-H-R-A-W-L-C-D-R-D**
or
SEQ ID NO: 2
   **-G-C-T-H-E-C-V-Q-E-P-F-G-A-K-C-**
or both epitopes.

The sequence for affinity binding of human DBP:VD complex seems to contain
SEQ ID NO: 03
   **-C-V-Q-E-P-**
or
SEQ ID NO: 04
   **-I-H-R-A-W-**

Other useful DBP binding epitopes within the megalin M2 region (E1024-K1429) are
SEQ ID NO: 05 (C19 sequence)
   **-S-D-F-N-G-G-C-T-H-E-C-V-Q-E-P-**
SEQ ID NO: 06 (D5 sequence)
   - **C-Y-N-M-R-G-S-F-R-C-S-C-D-T-G**
SEQ ID NO: 07 (A2 sequence)
   **-F-S-F-P-C-K-N-G-R-C-V-P-N-Y-Y**
as determined by the interaction of DBP with megalin M2 (E1024-K1429) - SEQ ID NO: 09 -using CelluSpots^{™} peptide array.

Preferred embodiments may comprise a determining of the megalin-bound complex of DBP:VD metabolites. This may be done using an antibody recognizing the complex of DBP:VD or after isolation of DBP, vitamin D or its metabolites or megalin or the soluble fragment thereof. In one aspect of the disclosure, the method may comprise providing mixtures of vitamin D metabolites and DBP for establishing standard samples.

In a preferred embodiment, the disclosure may comprise contacting said sample with an amount of added DBP to create standard conditions or a constant excess of DBP for a binding of the prohormone. The disclosure may further encompass contacting the sample with cubilin and/or a soluble fragment thereof.

Said embodiment may comprise a surface-bound DBP-binding megalin fragment or a fusion protein thereof which contains an epitope having any one or more of the amino acid sequence SEQ ID NO: 01 through SEQ ID NO: 10.

In one embodiment, the method of the disclosure may relate to a turbidimetric or nephelometric immunoassay. Said megalin and/or a soluble fragment thereof may be bound to nanoparticles having diameters ranging from 50 to 200 nm, so that the complex of DBP:VD is bound to said nanoparticles. The disclosure also provides an immunoassay selected from the group ELISA (enzyme-linked immunosorbent assay), RIA (radioimmunoassay), FIA (fluorescence immunoassay), L1A (luminescence immunoassay), or ILMA.

Consequently, the method may comprise the steps of (a) providing a defined amount of megalin and/or a soluble fragment thereof coupled to a solid phase; (b) contacting the sample with the solid phase having coupled megalin and/or a soluble fragment thereof; (c) creating conditions to allow binding of the complex formed by DBP and vitamin D metabolite, wherein DBP alone does not bind to megalin and/or a soluble fragment thereof; and washing the solid phase; (e) providing an antibody recognizing the ternary complex comprising DBP, vitamin D or its metabolite; (f) contacting said ternary complex with an antibody, optionally an antibody against DBP, and immobilizing the immunocomplex on the solid phase; and (g) determining the amount of antibody bound to the solid phase, and correlating the amount of bound antibody to the endocytic or activatable vitamin D status in blood, plasma, or serum by reference with a standard.

The instant disclosure further comprises a test kit for use in a method of measuring the effective vitamin D status which comprises an antibody specific for the ternary complex, megalin or a fragment thereof or DBP, or vitamin D and its metabolites. The disclosure also relates to a test kit for use in a method of measuring vitamin D metabolites in a sample of bodily fluid, comprising nanoparticles having bound megalin and/or soluble fragments thereof.

The achieved object is a simple and reliable method for a direct quantitative determination of the effective status of vitamin D and its metabolites in a sample. When the bodily fluid is blood, serum or plasma, the status describes the endocytable fraction of vitamin D metabolites in the circulation, say the fraction which can be processed innercellularly to the active hormone by respective target cells and tissues as needed. The novel vitamin D status can be determined in aqueous solution despite the highly lipophilic nature of vitamin D.

Vitamin D₃ is absorbed and processed by the organism easily and considered more potent than vitamin D₂ which has a shorter half life and binds with less affinity to the vitamin D receptor (VDR). 1α,25(OH)D₃ and parathyroid hormone (PTH) influence the vitamin D metabolism by positive or negative regulation of the activity of the la-hydroxylase and 24-hydroxylase (see Fig. 1C). Again, vitamin D is 25-hydroxylated in the liver to 25(OH)D₃ or 25(OH)D₂ , and further hydroxylated in kidney cells to its biologically active form 1a,25(OH)D. 24,25(OH)D is a further metabolite of 25(OH)D₃, but inactive and destined for excretion. In the circulation, vitamin D metabolites are tightly bound to DBP. Smaller amounts are bound to albumin and lipoproteins. The affinity of 25(OH)D (Ka = 6 x 10⁵ M⁻¹) and 1,25(OH)₂D (Ka = 5.4 x 10⁴M⁻¹) for albumin is substantially lower than the affinity for DBP (25(OH)D (Ka = 7 x 10⁸ M⁻¹) and 1,25(OH)₂D (Ka = 4 x 10⁷ M⁻¹)). Because of the abundance of albumin in serum (650 µM) compared to DBP (5 µM), some vitamin D will likely be bound by albumin while not effectively available for endocytosis. Additionally, the vast majority of the DBP in serum is not occupied by any vitamin D metabolite. There has been no disclosure in the prior art defining a parameter which addresses the bioavailabity of the vitamin D metabolites based on their binding to DBP and the following endocytic pathway. As DBP is present in high concentrations in the circulation it was so far not considered in the assessment of the vitamin D status. in the prior art, either total (bound and free) or free circulating vitamin D, separately, were considered sources of information for establishing a vitamin D status.

Megalin (also known as *Low Density Lipoprotein receptor-related Protein* 2, LRP2) is a multiligand binding cell receptor with structural similarities to the LDL receptor (LDLR). Megalin can be found in numerous cells and tissues, notably in the plasma membrane of absorptive epithelial cells (Farquhar MG et al, Soc. Nephrol. 6 (1): 35-47). LRP2/megalin is known to mediate the endocytosis of its ligands and can form complexes with cubilin which cubulin:megalin complexes are again able to (re)absorb molecules. The cubilin:megalin complex is inter alia responsible for the cellular uptake of lipids, VLDL, certain proteins (albumin, lactoferrin), cobalamin (vitamin B₁₂), and calcidiol. The instant disclosure proposes determining the amount of DBP:VD which is bound by megalin. This can be done for example by an ELISA against human DBP after "isolation or separation" of said ternary complex. This is preferred and the ELISA is already commercially available (Immundiagnostik AG, Bensheim). The term "effective vitamin D" describes formed complex of DBP:VD which has been bound by megalin (or a fragment thereof). The complex with megalin is considered to interact with cubulin so that the "activated vitamin D metabolite" will be endocytosed and subsequently 1a-hydroxylated to become the active hormone.

The effective vitamin D status represents an improvement over the prior art since it offers physiological information and a reading of the concentration of circulating vitamin D that will be processed to the D hormone. This status is different to the major circulating storage form because less active forms such as 25(OH)D₂ will not or much less contribute to the effective vitamin D status. The present disclosure provides a vitamin D status based on the amount DBP:VD, selectively and discriminably bound by megalin and/or cubulin. This endocytic complex therefore corresponds to the status of the effective vitamin D in serum or plasma. The term "bioavailable" as used in the prior art however refers to "total free" vitamin D which is speculative and is based on an assumed diffusion across the plasma membrane of cells.

There are contradictory views in the prior art with respect to the role of DBP. On the one hand, binding of vitamin D to DBP has been regarded a protection mechanism against excessive amounts of the free vitamin. This view is followed by groups supporting the "free hormone hypothesis". On the other hand, studies in DBP knockout mice showed that these animals have significantly reduced plasma levels of 25(OH)D₃ and 1,25-(OH)₂D₃. On a vitamin D depleted diet, the animals suffered from vitamin D deficiency and bone formation defects. These results point to the development of alternative pathways which will not be relevant when DBP is present in the circulation.

Steroid hormones and sterols such as vitamin D are lipophilic and commonly require a carrier protein for effective delivery. There are many ligand-specific serum carriers of steroid hormones and sterols including corticosteroid-binding globulin (CBG) (glucocorticoids, mineralocorticoids), vitamin A (retinol)-binding protein, vitamin D-binding protein (DBP), sex hormone-binding globulin (SHBG) (estrogens, androgens), and thyroid hormone-binding globulin. For example, CBG and SHBG not only act as high affinity serum transporters, but also bind to cell membranes in their ligand forms, suggesting alternative actions as signal transducers. In a similar fashion, DBP is a macrophage-activating factor (MAF) and actin-binder, which functions seem independent from the binding to vitamin D metabolites. The mechanisms by which ligands are released from binding globulins and acquired by target cells are crucial to steroid hormone signaling pathways.

This is particularly important for vitamin D where there is increasing evidence for extra-renal intracrine conversion of the pro-hormone (25(OH)D) to active D-hormone. The impact of vitamin D is then very much dependent on tissue-specific expression of the 1α-hydroxylase and the receptor for 1,25(OH)₂D, the nuclear vitamin D receptor (VDR). It has been estimated that concentrations of free 1,25(OH)₂D in serum are approximately 10⁻¹³ M, which is much less than the concentrations quoted for binding to the vitamin D receptor (dissociation constant (Kd) = approximately 10⁻¹⁰ M). Due to the obvious disparity between the amounts of free hormone available for (supposedly) passive diffusion and the levels required to efficiently occupy intracellular target receptors, the 'free-hormone hypothesis' raises doubts as a basis for a physiologically relevant ("true") vitamin D status.

Total 25(OH)D is currently measured by LC-MS/MS (*Institute of Standards and Technology and the Centers for Disease Control and Prevention*). A variety of immunoassays are used to determine concentrations of total 25(OH)D and other vitamin D metabolites. However, all these methods produce highly variable results, likely due to the need for a release of the vitamin D metabolites from their carrier proteins. This comprises a risk of a loss of vitamin D through binding to vessel surfaces so that a falsely (low) vitamin D level is determined. Affinity chromatography studies using immobilized DBP and solubilized rabbit kidney membranes have identified the co-receptors for the uptake of 25(OH)D₃-DBP complex in kidney tubules, namely a 600-kDa protein (megalin) and a 460-kDa protein (cubilin). These studies show a Ca²-dependent binding of DBP to cubilin. The authors do not suggest that the binding of DBP to either co-receptor is preceded, influenced or dependent on a formed complex of DBP and 25(OH)D₃, nor on the type of vitamin D metabolite bound by DBP (Nykjaer et al, Cubilin dysfunction causes abnormal metabolism of the steroid hormone 25(OH) vitamin D3, Proc Natl Acad Sci U S A. 2001 Nov 20; 98(24): 13895-13900).

Megalin has three domains: a 4400 amino acid extracellular amino-terminal domain, a 22 amino acid transmembrane domain and a 213 amino acid carboxy-terminal cytoplasmic tail, indicating that it is a type I cell-surface receptor. The extracellular domain contains four cysteine rich clusters of LDLR type A (complement-type) repeats. The complement type repeat consists of approximately 40 amino acids containing six cysteine residues and the SDE (Ser-Asp-Glu) motif responsible for high-affinity binding of positively charged sequences in ligands for LDLR. The four cysteine-rich clusters are flanked by epidermal growth factor (EGF)-type repeats and spacer regions containing YWTD (Tyr-Trp-Thr-Asp) motifs which are responsible for pH-dependent dissociation of ligands in endosomal compartments. The cytoplasmic domain of megalin contains three tetra-amino-acid NPXY motifs, which are essential for endocytosis of the ligand-receptor complex via clathrin-coated pits.

Megalin has diverse types of ligands: vitamin-binding proteins and other binding proteins, apolipoproteins, hormones and hormone precursors, drugs and toxins, enzyme and enzyme inhibitors, immune- and stress-response-related proteins, and others including calcium. Megalin knockout mice are unable to recover DBP from the glomerular filtrate, and lose it together with its vitamin D cargo in urine. As a consequence, megalin knockout mice are unable to adequately metabolize 25(OH)D to 1,25(OH)₂D resulting in a bone phenotype that resembles vitamin D-deficient rickets.

Cubilin has been identified as a receptor for intrinsic factor-B12 (IF-B) complex in the terminal ileum. It is a 460 kDa receptor with no transmembrane domain and no signals for endocytosis. Cubilin contains 27 CUB domains responsible for the ligand binding and eight EGF-type repeats preceded by a stretch of 110 amino acids, where the N-terminal region appears essential for membrane anchoring. A direct association between cubilin and megalin has been demonstrated, whereby molecular cooperation provides the basis for internalization of ligands bound to cubilin. Thus, cubilin binding ligands may undergo megalin-mediated endocytosis, unload its cargo in lysosomes and recycle back to the plasma membrane together with megalin.

Although megalin-dependent uptake of DBP has a clear role in renal vitamin D endocrinology, it is not yet clear whether a similar mechanism is present in other vitamin D target tissues. Outside the kidney, megalin is expressed by several tissues including the placenta, mammary gland and parathyroid glands, which are known to have 1a-hydroxylase activity, suggesting an extra-renal DBP-megalin interaction.

The present disclosure describes a method of determining a vitamin D status based on the detection and quantitation of a complex comprising DBP, vitamin D and either one of megalin and cubulin or both. Different to the prior art, the present method requires no specific release of the vitamin D metabolites from DBP and only the fraction of vitamin D metabolites will be evaluated that is subject to endocytosis and activation. Without wishing to be bound by any theory, the measurement of DBP bound to megalin provides a parameter for endocytable vitamin D metabolites. The provided method can therefore be used to establish a vitamin D status which corresponds to the physiologically active and endocytable vitamin D concentration in serum or plasma.

With the present method, the various vitamin D metabolites, in particular 25(OH)D₃ can be discriminated as well as the changes in total serum concentrations of 25OHD, 1,25(OH)₂D and DBP in subjects following supplementation with either cholecalciferol (vitamin D₃) or ergocalciferol (vitamin D₂). Supplementation will no longer interfere with the measurement of active vitamin D molecules that will be subject to endocytosis and hydroxylation. The fraction of truly bioavailable vitamin D molecules can therefoe easily be discerned from the supplemented vitamin D₂ or vitamin D molecules bound by other proteins in the circulation.

Immunonephelometry can be used to measure the DBP concentration in a sample. Immunonephelometry quantifies the scattering of an incident light source by large soluble antigen-antibody complexes under conditions of a moderate excess of antibody. Under these conditions, the complexes form a stable lattice, and a direct linear relationship is established between an increasing concentration of antigen and the increase in scattered light intensity. Automated nephelometers provide sensitive and precise measurements of DBP concentration in a rapid manner and with minimal requirements for technical skill. These can be used in clinical chemistry laboratories to analyze DBP concentrations. In a preferred embodiment, only the ternary complex formed by DBP, vitamin D and megalin is recognized by the antibody.

In another aspect, immunoturbidimetry can be used to measure the DBP concentration in a sample. Turbidimetry is the process of measuring the loss of intensity of transmitted light due to the scattering effect of particles suspended in it. Light is passed through a filter creating a light of known wavelength which is then passed through, for example, a cuvette containing a solution. A photoelectric cell collects the light which passes through the cuvette. A measurement is then given for the amount of absorbed light. Immunoturbidimetry is a variant in which an antigen-antibody reaction takes place. The antigen-antibody complexes are particles which can be optically detected by a photometer. In a preferred embodiment, the antibody recognizes a ternary complex formed by DBP, vitamin D and megalin. In another aspect, nanoparticles coated with megalin or fragments thereof are contacted with a sample containing DBP/vitamin D complexes. Increasing concentrations of DBP/vitamin D/megalin ternary complex in the sample result in increased turbidity.

### EXAMPLES

### EXAMPLE 1

Complex biological functions emerge through intricate protein-protein interaction networks. An important class of protein-protein interaction corresponds to peptide-mediated interactions, in which a short peptide stretch from one partner interacts with a large protein surface from the other partner. Protein-peptide interactions are typically of low affinity and involved in regulatory mechanisms, dynamically reshaping protein interaction networks. Due to the relatively small interaction surface, modulation of protein-peptide interactions has been considered feasible and highly attractive, for example, for therapeutic purposes. Unfortunately, the number of available 3D structures of protein-peptide interfaces is very limited. However, there was limited or no information regarding the interaction of DBP with other proteins, in particular, megalin/LRP2 (UniProtKB/Swiss-Prot, protein accession number P98164, human) in the context of vitamin D transport and metabolism. In order to examine the potential interaction of DBP with megalin at the peptide level, the PepSite2 program (hftp://pepsite2.russelllab.org) was used to predict peptide-binding spots. The PepSite method relies on preferred peptide-binding environments calculated from a set of known protein-peptide 3D structures, combined with distance constraints derived from known peptides. According to the Pepsite prediction, megalin likely interacts with human vitamin D-binding protein through ligand-binding repeats 9, 13-15, 16, 19, 22-25; 27 and 29 located at domains 2 and 3 (see **Figs. 1A**)

In agreement with the above prediction, the present inventors selected cysteine-rich complement-type ligand binding repeats (LDLR class A) for analysis of DBP-megalin interaction. EGF-like modules were also included as they are considered important for receptor folding and dissociation of ligands in endosomal compartment. A signal peptide (M1-G25) was introduced at the N-termini of complement-type repeats to allow sorting to the secretory pathway. A C-terminal 6xHis-tag was added for affinity purification and protein analysis by co-immunoprecipitation and Western blotting. The respective cDNA sequences were obtained by PCR and inserted into the mammalian expression vector pcDNA3.1. Since calcium is required for a correct folding of the LDLR-A domain, HEK cells were stable transfected with megalin cDNA sequences (M1, M2, M3) as they have endogenous calcium channels. Protein purification was done using the His-tag and a commercially available Ni-NTA resin. The cloning strategy for megalin fragments (M1, M2, M3) into the mammalian expression vector pcDNA3.1 is shown in **Figs. 2A-B.**

Megalin M1 cDNA encoded the sequence of amino acids 26 to 386 of human LRP2/megalin (SEQ ID NO:08). The construct included cDNA encoding for an N-terminal 25 amino acid signal peptide and a C-terminal 6x histidine tag. The predicted molecular weight was about 43 kDa for the monomer and about 86 kDa for the dimer.

The HEK-expressed and secreted recombinant megalin M1 fragment had the following amino acid sequence (without signal sequences):
SEQ ID NO: 08

Megalin M2 cDNA encoded the sequence of amino acids 1024-1429 of human LRP2/megalin (SEQ ID NO:09). The construct included cDNA encoding for an N-terminal 25 amino acid signal peptide and a C-terminal histidine(6x) tag. The predicted molecular weight was about 50 kDa for the monomer and about 100 kDa for the dimer. The HEK-expressed and secreted recombinant protein megalin M2 fragment had the following amino acid sequence (without signal sequences)::
SEQ ID NO: 09

Megalin M3 cDNA encoded the sequence of amino acids 2698 to 3192 of human LRP2/megalin (SEQ ID NO: 10). The cDNA encoded an 25 amino acid signal peptide and a C-terminal 6x histidine tag. The predicted molecular weight was about 60 kDa for the monomer and 120 kDa for the dimer. The construct was expressed in HEK 293 mammalian cells. The secreted megalin M3 fragment. SEQ ID **NO.10** corresponds to following amino acid sequence (without signal sequences):

Protein analysis of megalin M1, M2, M3 fragments was done by Western blotting employing an antibody against 6x His-tag. Cells transfected with above constructs (M1, M2, M3) were lysed and the lysate analyzed for the proteins with a His-tag. Megalin fragments were also purified from the cell supernatants using Ni/NTA resin, which bind proteins with a His-tag (right). The results are shown in **Fig. 2C**.

The Western blots show that megalin M1, M2, M3 fragments were expressed and secreted by HEK cells. The megalin fragments could be kept in solution. No precipitation was observed. The megalin fragments showed a tendency to form dimers but the molecular weights were all consistent with the predicted sizes. 2X Laemmli and 2X Urea sample buffers were used for analysis of megalin monomer and dimer formation but no difference was observed by the use of these buffers. The M2 fragment was the one which could be mostly easily dissociated to the monomer.

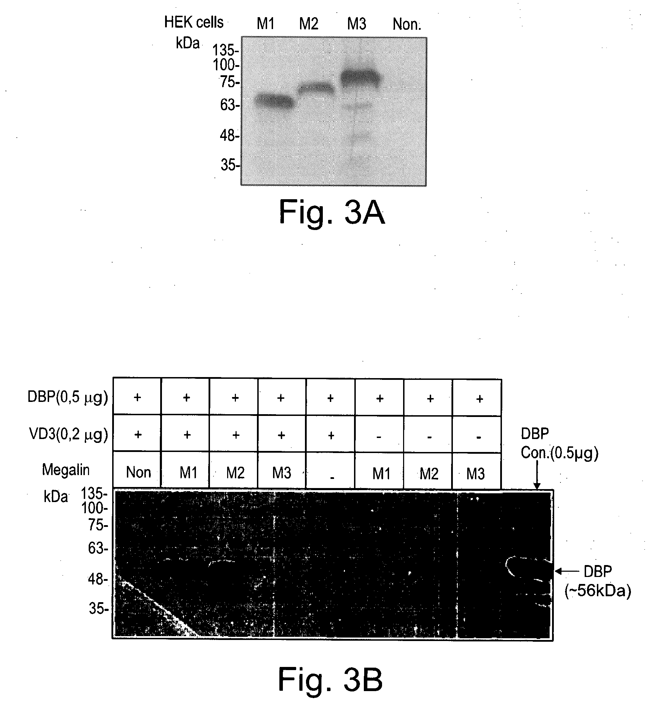

The megalin fragments were further examined for their interaction with DBP in the presence (+) or absence (-) of vitamin D₃ (VD₃). Co-immunoprecipitations were performed and the results are shown in **Figs 3** **A,B.** In brief, HEK cells were transfected with above cDNA constructs (M1, M2, M3) and cell lysates used for co-immunoprecipation. **Fig. 3A** shows a Western blot of cell lysates (20 µg) of HEK cells expressing M1, M2 and M3. Detection with an antibody against the His-tag. **Fig. 3B** shows the results of a co-immunoprecipitation with DBP (0,5 µg and VD₃ (0,2 µg).

The serum samples included DBP alone or in the presence of added vitamin D₃. Each sample was contacted with cell lysate containing megalin fragment. After incubation, the samples were contacted with beads coated with an antibody against His-tag and the DBP pulled down by centrifugation. The bound ligands (DBP) were dissociated from the beads and analyzed by Western blotting for the expression of DBP using an anti-DBP-antibody. As control, pure DBP alone was analyzed. **Fig. 3B** confirms that DBP could be pulled down with megalin M1 and M2 fragments. Notably, DBP could only be pulled down in the presence (+) of vitamin D₃. This indicates that the megalin fragments M1 and M2 do not interact with DBP alone but only when the DBP is occupied with a vitamin D metabolite. In other words, a ternary complex comprising vitamin D binding protein, vitamin D metabolite and megalin fragment was formed *in vitro,* and no interaction took place between megalin M1 or M2 fragments and DBP alone.

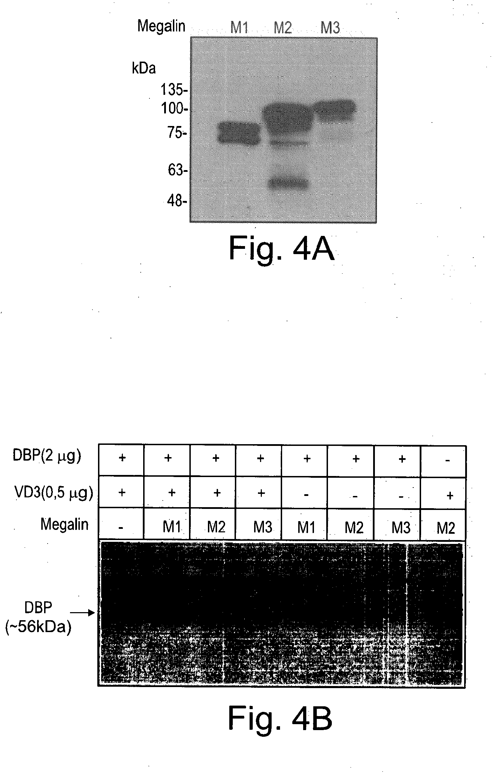

Additional co-immunoprecipitations show the *in vitro* interaction between DBP and Ni-NTA agarose purified soluble megalin M1, M2, M3 fragments (3 µg) in a similar set-up as above. The results are shown in **Figs. 4** **A,B.** **Fig. 4A** is a Western blot of purified megalin M1, M2, M3 fragments from culture supernatants (3 µg), secreted by HEK cells transfected with cDNA for megalin M1, M2 and M3 fragments; detection by anti-His-antibodies. **Fig. 4** **B** shows a western blot with the results of a co-immunoprecipitation with purified megalin M1, M2, M3 fragments. The serum samples contained DBP alone or in the presence (+) of vitamin D₃. After incubation, the samples were contacted with beads coated with an antibody against 6xHis-tag and pulled down by centrifugation. Bound proteins were dissociated from the beads and analyzed by Western blotting using an antibody against vitamin D binding protein. Again, **Fig. 4B** confirms that DBP could be pull down with purified soluble M1 and M2 fragments. DBP was pulled down only in the presence (+) of vitamin D₃. This indicates that soluble purified megalin fragments M1 and M2 do not interact with DBP unless occupied with vitamin D₃. Ternary complexes comprising DBP, vitamin D₃ and purified megalin M1 or M2 fragments were formed *in vitro.* No interaction or binding was observed between soluble purified megalin M1 or M2 fragments and DBP alone.

### EXAMPLE 2

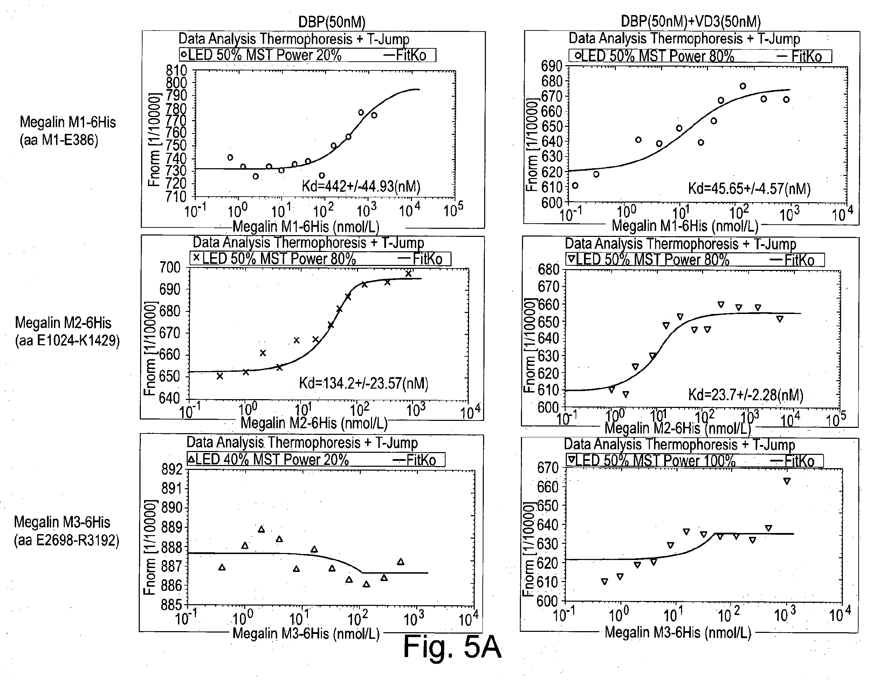

For further characterization of the formed complex of purifed soluble megalin M1 or M2 fragments and DBP alone or occupied by vitamin D metabolite, the obtained complexes were further analyzed by microscale thermophoresis and results are shown in **Fig. 5A****.** Microscale thermophoresis (MST) examines the directed movement of particles in a microscopic temperature gradient (thermophoresis). Any change of the hydration shell of biomolecules due to changes in their conformation results in a relative change of the movement along the temperature gradient. This principle can be used to determine the binding affinity of two molecules. This technique allows in particular an examination of interactions in solution without any immobilization on a surface. A spatial temperature difference leads to a depletion of molecule concentration in the region of elevated temperature, which can be then determined. Thermophoresis is usually performed with fluorescently labeled molecules.

The difference in the molecule's thermophoresis can further be used to quantify the binding strength under constant buffer conditions. The thermophoretic movement of the fluorescently labeled molecule is measured by monitoring the fluorescence distribution inside a capillary. The microscopic temperature gradient is generated by an IR-Laser, which is focused into the capillary and absorbed in water. The temperature of the aqueous solution in the laser spot region therefore increases. A homogeneous fluorescence distribution is observed inside the capillary prior the IR-Laser is switched on. When the IR-Laser is switched on, a new fluorescence distribution is established. The thermal relaxation time is fast and induces a binding-dependent drop in the fluorescence of the dye due to its local environmental-dependent response to the temperature step increase. Molecules move then from the locally heated region to the outer cold regions. The local concentration of molecules decreases in the heated region until it reaches a steady-state distribution.

The normalized fluorescence (Fnorm) measures a concentration ratio, with consideration of the temperature step increase. Due to the linearity of the fluorescence intensity and the thermophoretic depletion, the normalized fluorescence from the unbound molecule and the bound complex superpose linearly. Quantitative binding parameters were obtained using serial dilutions of the binding substrate. By plotting Fnorm against the logarithm of the different concentrations of the dilution series, a sigmoidal binding curve is obtained. This binding curve can directly be fitted with the non-linear solution of the law of mass action, with the dissociation constant Kd as result.

In brief, purified DBP was labeled with the red fluorescent dye NT-647 using Monolith Protein Labeling Kit Red (NanoTemper Technologies, Munich, Germany). Ni-NTA-purified soluble megalin fragments M1, M2, and M3 fragments were titrated in the range from 0.488 to 1000 nmol/L. A Monolith NT.115 device (NanoTemper Technologies) and the NT Analysis software version 1.427 (NanoTemper Technologies) were used for measurements.

Microscale thermophoretic analysis of the binding between His-Tag purified soluble megalin M1, M2, and M3 fragments and DBP in the absence or in the presence of vitamin D₃ were performed. The results are shown in **Figure 5A****.** In line with the above co-immunoprecipitation experiments, M1 and M2 fragments interacted with DBP but M3 did not. The dissociation constants were calculated for each experiment. Both megalin M1 and M2 fragments showed weak binding to DBP in the absence of 25-OH vitamin D₃ (M1/DBP: Kd= 442 +/- 44.93 nM; M2/DBP: 134.2 +/- 23.57 nM)). In contrast, high binding affintiy was found for both megalin fragments in the presence of 25(OH)D₃ as the dissociation constant Kd was markedly lower (M1/DBPIVD₃: Kd= 45.65 +/- 4.57 nM; M2/DBP/VD₃: 23.7 +/- 2.28 nM). We also observed increased binding of DBP to purified soluble megalin fragments M1 or M2 in the presence of 25(OH)D.

The result are good evidence of a 25(OH)D₃-dependent binding DBP to megalin. Megalin M2 fragment had a higher affinity for the complex of DBP: 25(OH)D₃ and was used therefore in further studies.

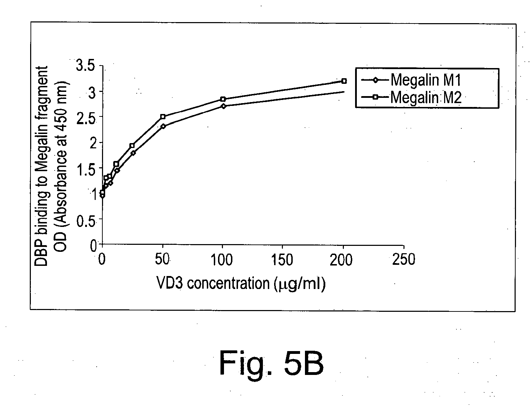

The impact of the 25(OH)D₃ concentration on the binding of soluble megalin M1 and M2 fragment to DBP was further assessed using an ELISA which already represents an embodiment of the proposed new complex binding assay for the effective vitamin D status. The results are shown in **Fig. 5B****.** In brief, a microtiter plate was coated with purified soluble megalin M1 or M2 fragment (1 µg/100 µl PBS). Mixtures of DBP and 25(OH)D₃ were prepared using 1 µg DBP and serial dilutions of 25(OH)D₃ (VD₃) in PBS (0, 0.3125, 0.625, 1.25, 2.5, 5, 10, 20 µg). Each mixture was applied to the coated wells, incubated at physiological conditions to allow formation of a ternary complex and washed. Detection and quantification of DBP bound by megalin (soluble fragments M1 and M2) was carried out by using a polyclonal rabbit antibody against DBP and HRP-conjugated donkey anti-rabbit antibody. Absorbance was read at 450 nm.

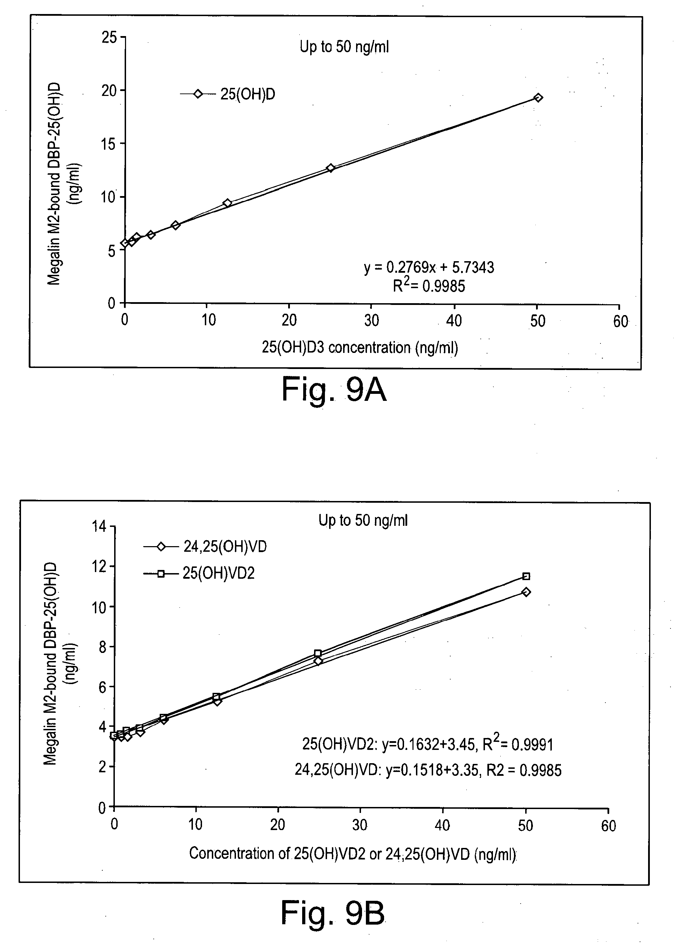

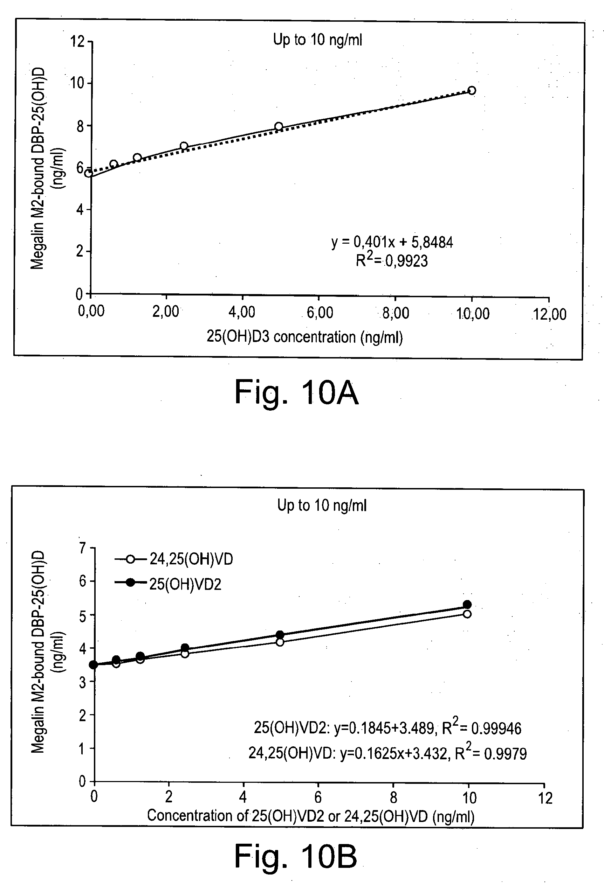

As shown in **Fig. 5B****,** the binding of soluble megalin M1 and M2 fragments to DBP was dependent on the 25-hydroxy-vitamin D₃. More precisely, the absorbance is linear proportional to the vitamin D₃ concentration as shown in **Fig. 9A** and **Fig. 10A** and this relation is not impacted by increasing concentrations of 25(OH)VDBP or 24,25(OH)VD (cf. **Fig. 9B****,** **10B).** Importantly, these experiments demonstrate a proof of principle for easy and reliable vitamin D measurements based on the ternary complex.

### EXAMPLE 3

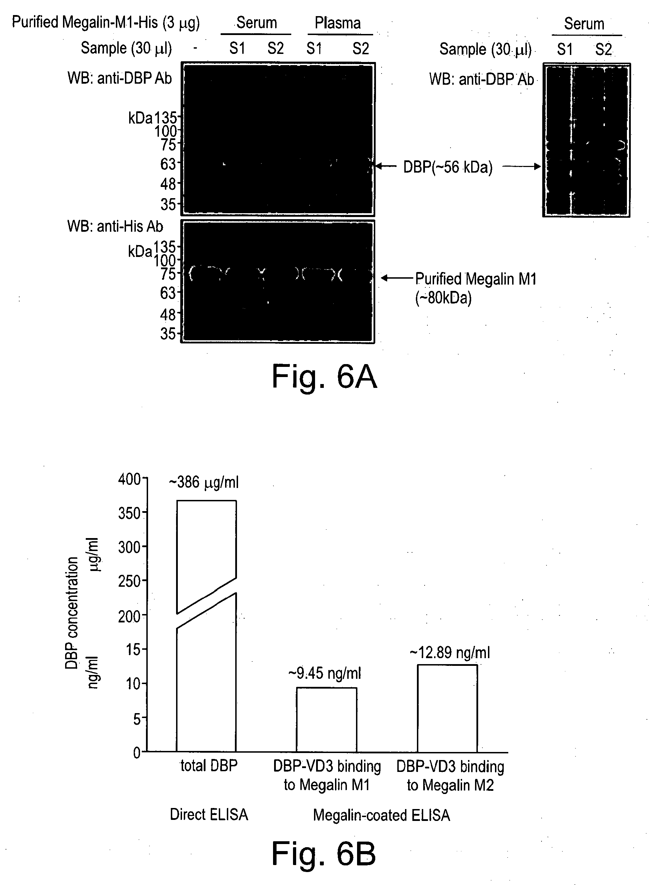

The binding properties of the various megalin soluble fragments were further analyzed by co-immunoprecipitation using samples of serum and plasma from human subjects. The results are shown in **Fig. 6A****.** Purified soluble megalin M1 protein was mixed with two human serum or plasma samples to allow interaction between soluble megalin M1 fragment and endogenous DBP present in the sample. Samples were incubated with Ni-NTA resin to allow interaction of megalin M1/DBP to the resin. After washing the resin, bound complex was eluted from the resin and assayed by western blotting. **Fig. 6A** (right) shows that DBP was present in both samples and could be detected using a specific antibody. **Fig. 6A** (left, upper blot) shows that purified soluble megalin M1 fragment interacted in solution with DBP in all samples, as demonstrated by the presence of pulled down DBP. Of note, using a control sample containing serum only but no megalin protein, no DBP was detectable. The presence of purified megalin M1 protein was detected in all samples using an anti-6xHis-tag antibody (**Fig. 6A****,** left, lower blot).

In summary, the co-immunoprecipitations are proof that the complex of DBP and 25(OH)D₃ can specifically be bound and isolated from plasma or serum using a suitable soluble megalin fragment. The megalin portion with amino acids 26-386 and 1024-1429 have been shown to be involved in the formation of a ternary complex with DBP and 25(OH)D₃. The external megalin region with amino acids 2698-R3192 did not participate in the binding under the described conditions.

The present application comprises representative amino acid sequences of megalin which can be used in the binding of DBP. Those can further be used for determining the status of endocytic or activatable vitamin D in bodily fluids. The status of endocytable vitamin D overrules any status for "free vitamin D" or "total vitamin D" as there will be no need for distinguishing between "free" or "total" from the "physiological status of vitamin D available for endocytosis and 1α-hydroxylation". Conventional methods usually do not determine the physiologically relevant vitamin D status since they cannot analyze the metabolites ready for processing to the active hormone. Thus, the conventional methods are insufficient whereas the present method is directed to the status of circulating prohormone which can and will be hydroxylated in the kidney, and probably in other tissues, giving rise to the active hormone.

### EXAMPLE 4

For detailed characterization of the proportion of the ternary complex of DBP , vitamin D₃ and megalin (M1 and M2 fragments) an ELISA was developed using megalin-coated plates. The total amount of DBP in the serum sample was determined using a commercial ELISA for DBP . The results are shown in **Figure 6B****.** The assay shows that a minor proportion (0.0028 %) only of the total amount of DBP in serum has bound hydroxylated vitamin D₃ and can be bound by megalin. Using megalin M1 fragment, the assay yielded a concentration value of 9.45 ng complex/mL serum. In the case of megalin M2 fragment, a value of about 12.89 ng complex/mL serum was obtained. This is evidence that the megalin fragment did only DBP when in a complex with the prohormone. Megalin can be thus regarded a means for detection and quantification of the physiologically activated prohormone (bound to DBP ) in a clinical sample.

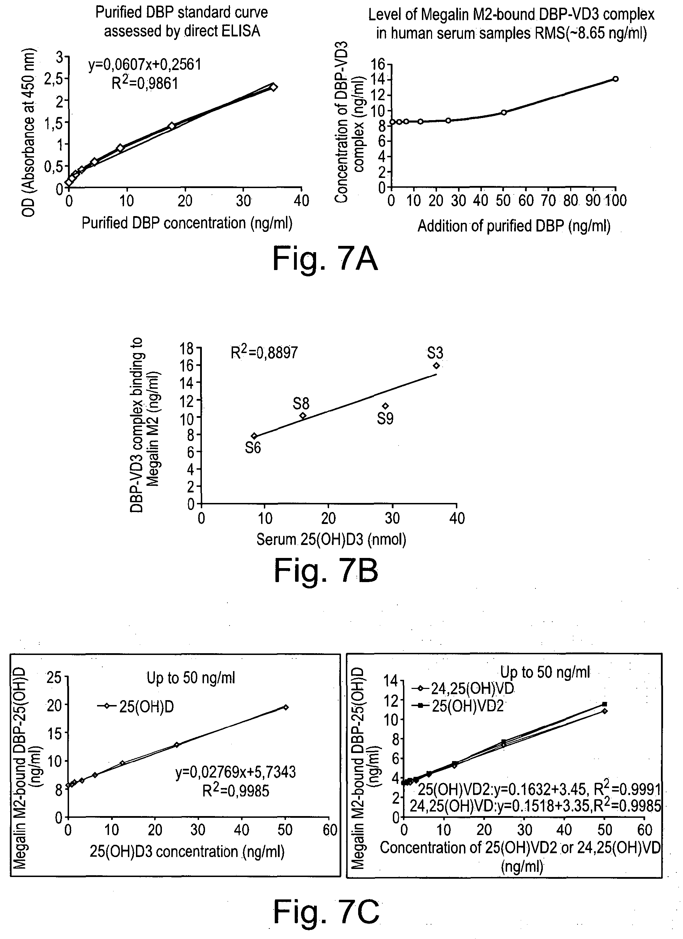

We noted that it was sometimes necessary to add an amount of purified DBP (DBP, from about 5 to 25 ng/mL) to induce a ternary complex between megalin, DBP and vitamin D metabolite in human serum. In order to assess the effect of added DBP on the formation of ternary complex we also performed ELISAs with increasing amounts of purified vitamin D binding protein. The results are shown in **Fig. 7A**. In the serum sample (RMS), addition of purified DBP in a dose range up to 25 ng/mL did not affect the binding of the complex DBP/VD₃ to purified soluble megalin M2 fragment. The initial value of 8.65 ng/mL remained unchanged independently of added purified DBP. A slight increase in complex-megalin interaction was observed when adding high amounts of purified DBP up to 40 ng/mL. Above this limit, the ternary complex was formed exponentially. Within limits, the measured levels of ternary complex of DBP-VD₃-megalin do therefore not depend on the amount of added purified vitamin D binding protein.

For comparison, five serum samples (S3, S6, S8, S9, RMS) from different subjects were analyzed to determine the concentration of total DBP (DBP), 25(OH)D₃ and ternary complex of DBP:25(OH)VD:M2. DBP was determined by ELISA. The 25(OH)D₃ content was quantified conventionally by an independent laboratory. The ternary complex DBP:25(OH)VD:M2 megalin was analyzed by a DBP:VD3:megalin ELISA according to the disclosure. **Table 1** below summarizes the results for comparison.

**TABLE 1**

| Serum sample | Total DBP (µg/mL) | 25(OH)VD₃ (nmol/L) / (ng/mL) | | DBP:25(OH)VD:M2 (ng/mL) |
|---|---|---|---|---|
| S3 | 576.7 ± 69.8 | 92 | 36.86 | 15.9 ± 2.4 |
| S6 | 208.1 ± 36.7 | 21 | 8.4 | 7.8 ± 1.1 |
| S8 | 352.2 ± 65.3 | 40 | 16 | 10.2 ± 1.4 |
| S9 | 732.5 ± 83.9 | 72 | 28.84 | 11.3 ± 1.8 |
| RMS | 424.9 ± 53.9 | - | - | 8.6 ± 1.5 |

Current standards for healthy control (interquartile) range values are for i) serum DBP 193.5-4345.0 g/ml (median 423.5 pg/ml; 354.1-586 pg/ml), and for ii) serum 25(OH)D3 30-100 ng/mL (normal range), 20-30 ng/ml (insufficiency), < 20 ng/ml (deficiency). Accordingly, sample S3 was considered to have normal levels of 25(OH)D₃; S9, insufficient; S8 and S6, deficient levels. In other words, from higher to lower levels of 25(OH)D₃: S3>S9>S8>S6. From the results it could be concluded that the level of total DBP seemed not to correlate with the level of 25(OH)D₃ level in human serum samples.

Although a direct comparison is strictly not possible since different analytes were measured by different methods, it is noteworthy that a correlation in the levels of ternary complex DBP:25(OH)VD:M2 megalin could be found in the samples when compared to 25(OH)D₃ values; from higher to lower levels of ternary complex S3>S9>S8>S6, identical as determined for 25(OH)D₃ levels. This further supports the notion that megalin only interacts *in vitro* with DBP when the prohormone is bound, providing a new status of physiologically active, endocytable vitamin D, which describes the bioavailability of the prohormone in the circulation.

The correlation between 25(OH)VD₃ serum levels and ternary complex was further analyzed; see graph in **Fig. 7B**. A linear correlation between serum levels of 25(OH)VD₃ and DBP:25(OH)VD:M2 megalin was determined (R²=0.8897) indicating that the method can provide accurate information that can be compared or related to standard values so as to obtain a measurement of the activatable vitamin D status of a subject.

Moreover, the linearity range of the interaction between megalin and DBP was examined using the claimed method for 25-hydroxyvitamin D₃ (25(OH)VD₃), 25-hydroxyvitamin D₂ (25(OH)VDBP) and 24,25-dihydroxyvitamin D (24,25(OH)VD). ELISAs for the complex of DBP:VD:megalin were performed as disclosed. In brief: a microtitre plate coated with purified megalin M2 fragment was incubated with a mixture of DBP and serial dilutions of 25(OH)VD₂, 25(OH)VD₃ or 24,25(OH)VD. For determination of the ternary complex, a polyclonal rabbit anti-DBP antibody and a HRP-conjugated donkey anti-rabbit antibody were used. The results are shown in **Fig. 7C**. For all vitamin D metabolites, the assay was linear up to 50 ng/ml. The determined sensitivity limit was 2.0 ng/ml.

The binding properties of DBP and 25(OH)VD₃, 25(OH)VD₂, 24,25(OH)VD and 3C-epimer of 25(OH)D₃ on the one hand and megalin on the other was further examined using microscale thermophoresis. Results are shown in **Figs. 8** **A,B.** The purified DBP was labeled with red fluorescent dye NT-647. The vitamin D metabolites were titrated with different concentrations. The dissociation constant Kd was calculated for every interaction partner. The binding affinity was assessed via the obtained dissociation constant Kd. The results show that the interaction affinity of DBP to 25(OH)VD₃ (Kd = 1.88 +/- 0.28 nM) or 25(OH)VDBP (Kd = 2.81 +/- 0.681 nM) were comparable, whereas the interaction to 24,25(OH)VD appeared to be slightly weaker. The 3C-epi25(OH)VD₃ bound most strongly.

The affinity of DBP when interacting with 25(OH)VD₃, 25(OH)VD₂, or 24,25-24,25(OH)VD, 3C-epimer of 25(OH)D₃ and megalin M2 was also analyzed by microscale thermophoresis. The results are shown in **Fig. 8B**. Purified DBP (50nM) was labeled with the red fluorescent dye NT-647 and mixed with either vitamin D metabolite (37.8 nM). Purified megalin shown in **Fig. 7C****.** For all vitamin D metabolites, the assay was linear up to 50 ng/ml. The determined sensitivity limit was 2.0 ng/ml.

The binding properties of DBP and 25(OH)VD₃, 25(OH)VD₂, 24,25(OH)VD and 3C-epimer of 25(OH)D₃ on the one hand and megalin on the other was further examined using microscale thermophoresis. Results are shown in **Figs. 8** **A,B.** The purified DBP was labeled with red fluorescent dye NT-647. The vitamin D metabolites were titrated with different concentrations. The dissociation constant Kd was calculated for every interaction partner. The binding affinity was assessed via the obtained dissociation constant Kd. The results show that the interaction affinity of DBP to 25(OH)VD₃ (Kd = 1.88 +/- 0.28 nM) or 25(OH)VDBP (Kd = 2.81 +/- 0.681 nM) were comparable, whereas the interaction to 24,25(OH)VD appeared to be slightly weaker. The 3C-epi25(OH)VD₃ bound most strongly.

The affinity of DBP when interacting with 25(OH)VD₃, 25(OH)VD₂, or 24,25-24,25(OH)VD, 3C-epimer of 25(OH)D₃ and megalin M2 was also analyzed by microscale thermophoresis. The results are shown in **Fig. 8B****.** Purified DBP (50nM) was labeled with the red fluorescent dye NT-647 and mixed with either vitamin D metabolite (37.8 nM). Purified megalin M2 was titrated in different concentrations. The dissociation constant Kd was calculated for every condition. The binding affinity was assessed by the dissociation constant Kd. The analyses show that the binding affinity of megalin M2 fragment to the complex DBP:25(OH)VD₃ is much stronger (Kd = 33.7 +/- 20.7 nM) than to the complex DBP:25(OH)VD₂ (Kd = 160 +/- 22.7 nM), or -/DBP-24,25VD (Kd = 171 +/- 24.6 nM). A very high affinity was observed for the 3C epimer which will require further investigation.

These experiments demonstrate that the binding of megalin to DBP takes only place when bound to the prohormone. This ternary complex can be detected and quantified by any method for determination of proteins. In view of the above, the use of megalin allows for a reliable measurement of the physiologically activated vitamin D, say 25-hydroxyvitamin D₃, as this represents the most active form of vitamin D in the circulation. The disclosed method provides for differential measurement of other vitamin D metabolites which is also relevant in view of the vitamin DBP in food supplements.

Megalin or fragments thereof are used to discern the effective vitamin D status. The described megalin fragments can easily be produced by recombinant methods and even by chemically synthesized. The described megalin fragments remained soluble in aqueous solution so that the conditions close to physiological can be used. There is no longer a need for a release of vitamin D from its binding partners, as required by the prior art methods. Thus, no solvents or surfactants for displacement of the vitamin D from its binding partners are needed. Accordingly, the measurement of the vitamin D status will not be interfered by non-physiological chemicals. No purification steps or time consuming and costly techniques such as LC-MS are necessary. A direct and fast determination of the effective vitamin D status can be done immediately after sample collection. The disclosed principles can further be easily adapted to available platforms and automats.

As other ligands than DBP can also bind to megalin, we have further mapped more closely the binding regions or epitope within the M2 region. The results are contained in the provided sequence for the binding epitopes. Minimum binding epitopes have further been tested.

### EXAMPLE 5

*Production of recombinant megalin fragments.* Megalin (LRP2) cDNA fragments were amplified by RT-PCR using mRNA from Caco-2 cells (human colon carcinoma epithelial cells). For cDNA transcription synthesis Maxima H Minus First strand cDNA synthesis kit (Thermo Scientific) was used. This kit allows synthess of cDNA up to 20 kb. Oligo dt18 primer and 65C were used. For PCR reactions Platinum PCR Supermix high fidelity PCR kit (Invitrogen) was used. Megalin M1 cDNA: 1158 bp; Megalin M2 cDNA: 1215 bp; Megalin M3 cDNA: 1482 bp.

Megalin cDNA fragments were cloned with a C-terminal His-Tag into pcDNA3.1 and the cloned plasmids transfected into mammalian HEK293 cells. The following cloning strategy was applied to clone megalin M2 cDNA encoding for sequence of amino acids 1024-1429 of human LRP2/megalin (SEQ ID NO:09), and resulted in the construct having: signal peptide (25 amino acids) + E1024-K1429 + 6x His tag. The cloning was done using PCR generated sequences.

Stable cell line selection was carried out using neomycin (G418, 800 µg/ml). Cell culture supernatants or cell lysates were purified by Ni-NTA resin. Analysis of megalin protein M1, M2, M3 fragments having a 6xHis-Tag was performed by Western blot with an antibody against 6xHis-tag (Cohesion Biosciences). Cell culture supernantants or cell lysates were purified with Ni-NTA agarose (Thermo Fischer Scientific). Cell lysates or culture supernatant (0.5 - 2 mL) were incubated with 50 µl of pre-equilibrated Ni-NTA resin at 4°C overnight. The resin was washed 3 times with H-buffer + 20mM imidazole. Megalin protein complex was eluted with H-buffer containing 200mM imidazole and buffer exchanged with PBS 1X. Protein solutions were lyophilized or kept at 4°C prior use.

The fragment M2 was further mapped for major binding epitopes of Megalin ligands within the M2 region, the region of megalin which binds human DBP. The binding sites of other megalin ligands were removed and the epitopes for binding of human DBP identified. It is imporant that the minimun epitopes do not overlap with the binding epitopes of other ligands off megalin. The results are shown in the Tables below.

**TABLE 2**

| *Major binding epitopes of Megalin ligands within M2 region (aa E1024-K1429)* | | |
|---|---|---|
| Human DBP | Epitope 1 | G-C-T-H-E-**C-V-Q-E-P**-F-G-A-K-C |
| | Epitope 2 | D-N-G-N-C-**I-H-R-A-W**-L-C-D-R-D |
| Rabbit-anti-hDBP-IgG Ab (Immundiagnostik) | A19-A20 | Y-T-C-D-N-H-Q-C-I-S-K-N- W-V -C-D- T -D-N-D |
| Human Ig κC | C8: | M-S-D-E-K-D-C-P-T-Q-P-F-R-C-P |
| Human C1q | A19 B22 | Y-T-C-D-N-H-Q-C-I-S-K-N-W-V-C E-C-D-G-H-P-D-C-L-Y-G-S-D-E-H |
| Human Albumin-His-tag | Epitope 1 (B22): | E-C-D-G-H-P-D-C-L-Y-G-S-D-E-H |
| | Epitope 2 (C24-D1): | F-L-L-A-N-D-S-K-T-C-E-D-I-D-E-C-D-I-L-G |
| Human RAP | Epitope 1 (B18-B19): | M-C-H-S-D-E-F-Q-C-Q-E-D-G-I-C-I-P-N-F-W |
| | Epitope 2 (D8): | C-D-T-G-Y-M-L-E-S-D-G-R-T-C-K |
| Human ApoE | Epitope 1(AH): | C-G-H-G-E-C-I-P-A-H-W-R-C-D-K |
| | Epitope 2 (D₃): | C-D-I-L-G-S-C-S-Q-H-C-Y-N-M-R |
| | Epitope 2 (D8): | C-D-T-G-Y-M-L-E-S-D-G-R-T-C-K |
| Human PTH | Epitope 1 (B22): | E-C-D-G-H-P-D-C-L-Y-G-S-D-E-H |

**TABLE 3**

| *Epitope mapping of ligands binding to the human Megalin M2 region (aa E1024-K1429)* | |
|---|---|
| LDL-receptor class A8 (1024-1061) | EQCGLFS FPCKNGRCVPNYYLCDGVDDCHDNSDEQLCG |
| LDL-receptor class A9 (1066-1102) | |
| LDL-receptor class A10 (1108-1144) | |
| LDL-receptor class A11 (1148-1184) | STETCQPSQFNCPNHRCIDLSFVCDG DKDCVDGSDEVGCV |
| LDL-receptor class A12 (1186-1223) | LNCTASQFKCASGDKCIGVTNRCDGVFDCSDNSDEAGCP |
| LDL-receptor class A13 (1229-1267) | |
| LDL-receptor class A14 (1270-1306) | |
| LDL-receptor class A15 (1304-1349) | |
| Special domain (1350-1389) | |
| EGF-like1; calcium-binding (1390-1429) | |

The minimum epitopes within the megalin M2 region for a bindung of human DBP have been listed in Table 3 below.

**TABLE 4**

| *List of Megalin-M2-peptides cloned and expressed as fusion polypeptides with furin* | | |
|---|---|---|
| **Name** | **Peptide Sequence** | **Peptide cDNA** |
| Peptide 1-15aa | DNGNCIHRAWLCDRD | |
| Peptide 2-15aa | GCTHECVQEPFGAKC | |
| Peptide 3-30aa | | |
| Peptide 4-30aa | | |

### EXAMPLE 6

*Megalin complex ELISA binding assay.* Microtiter plates were coated with soluble purified megalin M1 or M2 fragment (1 µg/100 µl diluted in PBS1X) by incubation at RT for 2 h. Serial dilutions of 25-hydroxvitamin D₃ (VD₃) 0, 0.3125, 0.625, 1.25, 2.5, 5, 10, 20 ng in 100 µl PBS were mixed each with 1 µg DBP (DBP). The mixture was incubated at 37°C for 1h. Unspecific binding sites were blocked with blocking buffer (1% BSA in PBS1X) at RT for 1 h. The DBP-VD₃ mixture or serum samples were added to the megalin-coated wells and incubated at 4°C overnight. After washing with PBST-buffer (0.05% Tween 20 in PBS) 100 µl rabbit-anti-DBP antibody was added (1: 3500 diluted in blocking buffer) and incubated at R.T for 2 h. After washing with PBST, 100 µl of HRP- conjugated donkey anti-rabbit 2nd antibody (1: 500 diluted in blocking buffer) was added and incubated at 37°C for 1 h. 100 µl substrate reagent A+B (1:1) (R&D) was added and incubated at R.T for 30min. Then, 100 µl stop solution was added to the wells. Absorbance was read at 450 nm. The values were compared to standard values of known 25-hydroxvitamin D₃ concentration.

### EXAMPLE 7

*Co-immunoprecipitation of DBP from human serum with recombinant purified soluble megalin.* Ni-NTA purified soluble megalin M1 or M2 protein fragment (3 µg) was first mixed with human serum or plasma samples (30 µl). These samples were incubated with 50 µl of pre-equilibrated Ni-NTA resin at 4°C overnight. The resin was washed 3 times with H-buffer + 20mM imidazole. Bound complex was eluted with H-buffer containing 200mM imidazole and analyzed by Western blot with a polyclonal rabbit antibody against DBP (Abcam).

### EXAMPLE 8

*Immunoturbidimetry assay for determination of vitamin D status with soluble megalin.* Ni-NTA purified soluble megalin M1 or M2 protein fragment (3 µg) is first mixed with human serum or plasma samples (30 µl). Samples are incubated with 50 µl of pre-equilibrated Ni-NTA resin at 4°C overnight. The resin is washed 3 times with H-buffer + 20mM imidazole. Bound complex is eluted with H-buffer containing 200 mM imidazole. The eluted complex is contacted in aqueous solution with an antibody against vitamin D binding protein. The increase in turbidity is measured with a standard turbidimeter and compared with standard values of known 25-hydroxvitamin D₃ concentration.

Alternatively, nanoparticles, for example, latex nanoparticles (aprox. 150 nm), are coated with megalin M1 or M2 fragment and incubated with a serum or plasma sample. The increase of turbidity is then measured and compared to standard values of known 25-hydroxvitamin D₃ concentration.

### EXAMPLE 9

*ELISA binding assay for linearity range determination.* A microtiter plate was coated with purified megalin M1 or M2 protein fragment (1 µg/100 µl diluted in PBS1X) by incubation at RT for 2 h. Mixtures of DBP and 25-hydroxvitamin D₃ (VD₃), 25-hydroxvitamin D₂ (VD₂) or 24,25-hydroxvitamin D (24,25VD) were prepared by mixing 20 µg DBP and serial dilution of VD (0, 0.78, 1,56, 3,125, 6.25, 12.5, 25, 50 ng) in 100 µl PBS. The mixture was incubated at 37°C for 1h. After plate blocking, the above prepared DBP-VD mixture was added to megalin-coated wells and incubated at 4°C, overnight. Then, the plate was incubated with 100 µl diluted rabbit-anti-DBP antibody (1:1000 diluted in blocking buffer) at RT for 2 h, followed by incubation with 100 µl of HRP-conjugated donkey anti-rabbit antibody (1: 500 diluted in blocking buffer) at 37°C, 1 h. After substrate reaction, the absorbance was read at 450 nm. The assay was linear up to 50 ng/ml. The sensitivity limit was determined to be 2.0 ng/ml.

### EXAMPLE 10

*Microscale Thermophoresis assay.* Purified DBP (Merck, 345802) was labeled with the red fluorescent dye NT-647 by using a Monolith Protein Labeling Kit Red (NanoTemper Technologies, Munich, DE). Ni-NTA-purified megalin fragments (M1, M2) were titrated in the range of 0.488 to 1000 nmol/L. Purified DBP (Merck, 345802) was likewas labeled with the red fluorescent dye NT-647 by using the Monolith Protein Labeling Kit Red. 25-hydroxvitamin D₃ (VD₃) was titrated in concentrations in the range of 0.0488 to 100 nmol/L. 25-hydroxvitamin D₂ (VD₂), 24,25-hydroxvitamin D (24,25VD) and 3epi25(OH)VD₃ were titrated in concentrations in the range of 0.0163 to 37 nmol/L. 37.8 nM of VD₃, VD₂ and 24,25VD was respectively added to 50nM of NT-647 labeled DBP. A Monolith NT.115 device (NanoTemper Technologies) was used for measurements. NT Analysis software version 1.427 (NanoTemper Technologies) was used for analysis. Parameters: laser power, 100%; LED, 80; laser on-time, 30 seconds; laser off-time, 5 seconds; temperature, 25°C. FNorm: normalized fluorescence; Kd: dissociation constant.

The dissociation constants Kd of the vitamin D metabolites to DBP gave the following ranking of the binding affinities. 3epi25(OH)₂VD3 > 25(OH)₂VD3 ≥ 25(OH)₂VD₂ > 24,25(OH)₂VD3 > 1,25(OH)₂VD3. The binding affinity of DBP-VD₂ is comparable to Kd of DBP-VD₃. The binding of 24,25(OH)VD to DBP is marginally lower. The high binding affinity of the 3C epimer of 25(OH)D₃ is surprising and will require further investigation as this epimer seems therefore particularly useful as food supplement, if not toxic for other reasons.

## Claims

1. A method of measuring vitamin D and its metabolites in a sample of bodily fluid from a subject, which sample may contain endogenous vitamin D binding protein (DBP), comprising the steps of:
a) contacting said sample with megalin and/or a fragment thereof under binding conditions to form a ternary complex comprising DBP, vitamin D metabolite and megalin or a fragment thereof;
b) determining the amount of DBP bound by megalin or a fragment thereof; and
c) relating the amount of megalin-bound DBP to the vitamin D status of said subject.

2. Method according to claim 1, wherein said fragment of megalin comprises any one of amino acid sequences of SEQ ID NO:01 through SEQ ID NO:10

3. Method according to claim 1 or claim 2, wherein said fragment of megalin comprises amino acid SEQ ID NO:1 or SEQ ID NO:2 or combinations thereof.

4. The method of any claim 1 to 3, wherein the fragment of megalin binds exclusively vitamin D binding protein (DBP), and none of the other ligands of megalin.

5. The method of any claim 1 to 4, comprising a determining of the bound vitamin D metabolite.

6. The method of any claim 1 to 5, comprising a differential measurement of 25-hydroxyvitamin D₃ in the presence of 25-hydroxyvitamin D₂, 24,25-dihydroxyvitamin D₂, or 24,25-dihydroxyvitamin D₃.

7. Method according to any claim 1 to 6, comprising the addition of a known amount of vitamin D binding protein to the sample.

8. Method according to any claim 1 to 7, further contacting said sample with cubilin and/or a soluble fragment thereof.

9. Method according to any claim 1 to 8, wherein said complex with DBP is determined by particle-enhanced immunoturbidimetry or nephelometry.

10. Method according to any claim 1 to 8, wherein said complex with DBP is determined by an immunoassay selected from the group ELISA (enzyme-linked immunosorbent assay), RIA (radioimmunoassay), FIA (fluorescence immunoassay), LIA (luminescence immunoassay), or ILMA.

11. Method according to any claim 1 to 10, comprising the steps of:
(a) providing a defined amount of megalin and/or a soluble fragment thereof coupled to a solid phase;
(b) contacting the sample with the solid phase having immobilized megalin and/or a soluble fragment thereof;
(c) creating conditions to allow binding of megalin and/or a soluble fragment thereof to the complex formed by vitamin D binding protein (DBP) and vitamin D metabolites, wherein vitamin D binding protein (DBP) alone does not bind to megalin and/or a soluble fragment thereof; and washing the solid phase;
(e) providing an antibody complex recognizing the complex comprising DBP, vitamin D or its metabolites and megalin or a soluble fragment thereof;
(f) contacting said megalin and/or a soluble fragment thereof bound to vitamin D binding protein (DBP) and vitamin D metabolites with the antibody against vitamin D binding protein (DBP), and immobilizing the complex on the solid phase; and
(g) determining the amount of antibody bound to the solid phase, and quantitating the vitamin D metabolites in blood plasma or serum by correlation with standard samples.

12. Test kit for use in a method of determining the vitamin D status in a sample of bodily fluid, comprising an antibody binding DBP and a fragment or amino acid sequence of megalin, optionally coupled to a carrier.

13. Composition for measuring vitamin D metabolites in a sample of bodily fluid, comprising antibodies against an epitope of DBP and/or a fragment of megalin.

## Patentansprüche

1. Verfahren zur Messung von Vitamin D und dessen Metaboliten in einer Probe mit Körperflüssigkeit des Probanden, wobei die Probe endogenes Vitamin-D-bindendes Protein (DBP) enthalten kann, umfassend die Schritte:
a) In-Kontakt-bringen der Probe mit Megalin und/oder einem Fragment davon unter Bindungsbedingungen, so dass man einen ternären Komplex erhält, der DBP, einen Vitamin-D-Metaboliten und Megalin oder dessen Fragment umfasst;
(b) Bestimmen der Menge an DBP, die von Megalin oder dessen Fragment gebunden wird; und
(c) Beziehen der Menge des an Megalin gebundenen DBP mit dem Vitamin-D-Status des Probanden.

2. Verfahren nach Anspruch 1, wobei das Fragment des Megalins eine der Aminosäuresequenzen von SEQ ID NO:01 bis SEQ ID NO:10 umfasst.

3. Verfahren nach Anspruch 1 oder Anspruch 2, wobei das Fragment des Megalins die Aminosäuresequenz SEQ ID NO:01 oder SEQ ID NO:02 oder Kombinationen davon umfasst.

4. Verfahren nach einem der Ansprüche 1 bis 3, wobei das Fragment des Megalins ausschließlich Vitamin-D-bindendes Protein (DBP) und keinen der anderen Liganden von Megalin bindet.

5. Verfahren nach einem der Ansprüche 1 bis 4, umfassend die Bestimmung von gebundenen Vitamin-D-Metaboliten.

6. Verfahren nach einem der Ansprüche 1 bis 5, umfassend eine differenziale Bestimmung von 25-Hydroxyvitamin D₃ in Gegenwart von 25-Hydroxyvitamin D₂, 24,25-Dihydroxyvitamin D₂ oder 24,25-Dihydroxyvitamin D₃

7. Verfahren nach einem der Ansprüche 1 bis 6, umfassend die Zugabe einer bekannten Menge an Vitamin-D-bindendem Protein zu der Probe.

8. Verfahren nach einem der Ansprüche 1 bis 7, wobei die Probe ferner mit Cubulin und/oder einem löslichen Fragment davon in Kontakt gebracht wird.

9. Verfahren nach einem der Ansprüche 1 bis 8, wobei der Komplex mit DBP durch partikelverstärkte Immunoturbidimetrie oder Nephelometrie bestimmt wird.

10. Verfahren nach einem der Ansprüche 1 bis 8, wobei der Komplex mit DBP bestimmt wird durch einen Immunoassay, ausgewählt aus der Gruppe mit ELISA (Enzyme-linked Immunosorbensassay), RIA (Radioimmunoassay), FIA (Fluoreszenz-Immunoassay), LIA (Lumineszenz-Immunoassay) oder ILMA,

11. Verfahren nach einem der Ansprüche 1 bis 10, das die folgenden Schritte umfasst:
(a) Bereitstellen einer definierten Menge von Megalin und/oder eines löslichen Fragments davon, gekoppelt an eine feste Phase;
(b) in Kontakt bringen der Probe mit der festen Phase mit immobilisiertem Megalin und/oder einem löslichen Fragment davon;
(c) Erzeugen von Bedingungen, die eine Bindung von Megalin und/oder einem löslichen Fragment davon an den durch das Vitamin-D-bindende Protein (DBP) und Vitamin-D-Metaboliten gebildeten Komplex ermöglichen, wobei das Vitamin-D-bindende Protein (DBP) allein nicht an Megalin und/oder ein lösliches Fragment davon bindet; und Waschen der festen Phase;
(e) Bereitstellen eines Antikörperkomplexes, der den Komplex aus DBP, Vitamin D oder dessen Metaboliten und Megalin oder einem löslichen Fragment davon erkennt; (f) in Kontakt bringen des Megalins und/oder eines löslichen Fragments davon, das an das Vitamin-D-bindende Protein (DBP) und die Vitamin-D-Metaboliten gebunden ist, mit dem Antikörper gegen das Vitamin-D-bindende Protein (DBP) und Immobilisieren des Komplexes an der festen Phase; und
(g) Bestimmung der Menge des an die feste Phase gebundenen Antikörpers und Quantifizierung der Vitamin-D-Metaboliten in Blutplasma oder Serum durch Korrelation mit Standardproben.

12. Testkit zur Verwendung in einem Verfahren zur Bestimmung des Vitamin-D-Status in einer Probe einer Körperflüssigkeit, umfassend einen Antikörper, der DBP bindet, und ein Fragment oder eine Aminosäuresequenz von Megalin, gegebenenfalls gekoppelt an einen Träger.

13. Zusammensetzung zur Messung von Vitamin-D-Metaboliten in einer Probe mit Körperflüssigkeit, die Antikörper gegen ein Epitop von DBP und/oder ein Fragment des Megalins enthält.

## Revendications

1. Procédé de mesure de la vitamine D et de ses métabolites dans un échantillon de fluide corporel du sujet, ledit échantillon pouvant contenir une protéine endogène de liaison à la vitamine D (DBP), comprenant les étapes consistant à :
(a) la mise en contact de l'échantillon avec la mégaline et/ou un fragment de celle-ci dans des conditions de liaison, de manière à obtenir un complexe ternaire comprenant la DBP, un métabolite de la vitamine D et la mégaline ou son fragment ;
(b) déterminer la quantité de DBP liée par la mégaline ou son fragment ; et
(c) relier la quantité de DBP liée à la mégaline au statut en vitamine D du sujet.

2. Procédé selon la revendication 1, dans lequel le fragment de mégaline comprend l'une des séquences d'acides aminés de SEQ ID NO:01 à SEQ ID NO:10.

3. Procédé selon la revendication 1 ou la revendication 2, dans lequel le fragment de mégaline comprend la séquence d'acides aminés SEQ ID NO :01 ou SEQ ID NO :02 ou des combinaisons de celles-ci.

4. Procédé selon l'une quelconque des revendications 1 à 3, dans lequel le fragment de mégaline se lie exclusivement à la protéine de liaison à la vitamine D (DBP) et à aucun des autres ligands de la mégaline.

5. Procédé selon l'une quelconque des revendications 1 à 4, comprenant le dosage des métabolites de la vitamine D liés.

6. Procédé selon l'une des revendications 1 à 5, comprenant un dosage différentiel de la 25-hydroxyvitamine D3 en présence de 25-hydroxyvitamine D2, de 24,25-dihydroxyvitamine D2 ou de 24,25-dihydroxyvitamine D3.

7. Procédé selon l'une quelconque des revendications 1 à 6, comprenant l'addition d'une quantité connue de protéine liant la vitamine D à l'échantillon.

8. Procédé selon l'une quelconque des revendications 1 à 7, dans lequel l'échantillon est en outre mis en contact avec la cubuline et/ou un fragment soluble de celle-ci.

9. Procédé selon l'une quelconque des revendications 1 à 8, dans lequel le complexe avec la DBP est déterminé par Immunoturbidimetrie renforcée par des particules ou par néphélométrie.

10. Procédé selon l'une quelconque des revendications 1 à 8, dans lequel le complexe avec le DBP est déterminé par un immunodosage choisi dans le groupe comprenant l'ELISA (enzyme-linked immunosorbent assay), le RIA (radioimmunoassay), le FIA (fluorescence immunoassay), le LIA (luminescence immunoassay) ou l'ILMA,

11. Procédé selon l'une quelconque des revendications 1 à 10, comprenant les étapes suivantes :
(a) fourniture d'une quantité définie de mégaline et/ou d'un fragment soluble de celle-ci, couplée à une phase solide ;
(b) mettre l'échantillon en contact avec la phase solide contenant de la mégaline immobilisée et/ou un fragment soluble de celle-ci ;
(c) créer des conditions permettant la liaison de la mégaline et/ou d'un fragment soluble de celle-ci au complexe formé par la protéine de liaison à la vitamine D (DBP) et le métabolite de la vitamine D, la protéine de liaison à la vitamine D (DBP) seule ne se liant pas à la mégaline et/ou à un fragment soluble de celle-ci ; et laver la phase solide ;
(e) fournir un complexe d'anticorps qui reconnaît le complexe de DBP, de vitamine D ou de ses métabolites et de mégaline ou d'un fragment soluble de celle-ci ; (f) mettre en contact la mégaline et/ou un fragment soluble de celle-ci lié à la protéine de liaison à la vitamine D (DBP) et aux métabolites de vitamine D avec l'anticorps dirigé contre la protéine de liaison à la vitamine D (DBP) et immobiliser le complexe sur la phase solide ; et
(g) déterminer la quantité d'anticorps lié à la phase solide et quantifier les métabolites de la vitamine D dans le plasma sanguin ou le sérum par corrélation avec des échantillons standard.

12. Trousse d'essai destinée à être utilisée dans une méthode de détermination du statut en vitamine D d'un échantillon de fluide corporel, comprenant un anticorps qui se lie à la DBP et un fragment ou une séquence d'acides aminés de mégaline, éventuellement couplés à un support.

13. Composition pour la mesure des métabolites de la vitamine D dans un échantillon de fluide corporel contenant un anticorps dirigé contre un épitope de DBP et/ou un fragment de mégaline.
